(19) Europäisches Patentamt
European Patent Office
Office européen des brevets

(11) Publication number: 0 191 860 B1

(12) # EUROPEAN PATENT SPECIFICATION

(45) Date of publication of patent specification: 18.04.90

(51) Int. Cl.$^5$: C 09 K 19/34, C 07 D 239/26, C 07 D 239/28, C 07 D 239/34

(21) Application number: 85902658.5

(22) Date of filing: 30.05.85

(86) International application number: PCT/JP85/00302

(87) International publication number: WO 86/00087 03.01.86 Gazette 86/01

(54) **LIQUID CRYSTAL COMPOUND.**

(30) Priority: 07.06.84 JP 117209/84
09.07.84 JP 141700/84
11.07.84 JP 144027/84
15.10.84 JP 215367/84
27.11.84 JP 250171/84
27.11.84 JP 250172/84
04.03.85 JP 42116/85
04.03.85 JP 42117/85
20.03.85 JP 56652/85

(43) Date of publication of application: 27.08.86 Bulletin 86/35

(45) Publication of the grant of the patent: 18.04.90 Bulletin 90/16

(84) Designated Contracting States: CH DE FR GB LI SE

(56) References cited: No relevant documents have been disclosed.

(73) Proprietor: SEIKO INSTRUMENTS INC.
31-1, Kameido 6-chome Koto-ku
Tokyo 136 (JP)

(73) Proprietor: TEIKOKU CHEMICAL INDUSTRY CO., LTD.
1-18, 1-chome Kitahorie
Nishi-ku Osaka (JP)

(72) Inventor: TAGUCHI, Masaaki
31-1, Kameido 6-chome
Koto-ku Tokyo 136 (JP)
Inventor: HARADA, Takamasa
31-1, Kameido 6-chome
Koto-ku Tokyo 136 (JP)
Inventor: SUENAGA, Hitoshi Itami Factory,
Teikoku Chemical
Industry Co., Ltd. 41, Senzo 5-chome
Itami-shi Hyogo 664 (JP)

(74) Representative: Miller, Joseph et al
J. MILLER & CO. Lincoln House
296-302 High Holborn
London WC1V 7JH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to new liquid crystal compounds useful in electric optical devices, utilizing the response of a ferroelectric smectic liquid crystal to an electric field.

Liquid crystals are already used in many types of electric optical devices such for example as display devices, shutter alleys for printers, shutters for cameras, and displays in watches and calculators. Most of the liquid crystal display elements presently used are elements utilizing the dielectric orientation efficiency of a nematic liquid crystal or a cholestric liquid crystal. However, with regard to the expected adaptation of the display elements with many picture elements, there are such problems as the response ability being insufficient, the contrast not being able to obtain the drive margin, and the visual angle characteristic being insufficient. Therefore, extensive research and development is being carried out with respect to the TFT panel or the MOS panel that forms the switching element in each picture element.

U.S.A. Patent Specification No. 4,367,924 discloses a liquid crystal element with a new display principle, which utilizes the smectic phase and which obviates or reduces the above defects of the liquid crystal element. An explanation of this new liquid crystal element will now be given.

The liquid crystal is composed of molecule layers and in each of the layers, the average direction of the molecule axis is inclined 40 degrees in a vertical direction to the layers. Meyer et al teaches in a thesis entitled "Ferroelectric liquid crystal" in "Physical Journal" (Le Journal de Physique Vol. 36, March, 1975 PPL-69 to L-71) that, the smectic C* phase or the smectic H phase is composed of an optically active molecule which generally has an electric dipole density P and is ferroelectric. This dipole density P is vertical to the molecule direction n, and is parallel to the layer surface of the smectic. What Meyer et al teach is adaptable in smectic H phase, but in the smectic H phase, the viscosity to the rotation around the axis vertical to the layer, becomes large. The existence of electric dipoles in the chiral smectic liquid crystal gives a stronger coupling force to the electric field, than that of dielectric anisotropy. Furthermore, this coupling force has a polarity characteristic in the sense that a favourable direction of P is the parallel direction to electric field E, and so, by reversing the direction of the applied electric field, the direction of P can also be reversed.

Therefore, by reversing the electric field, the direction of the molecule can be controlled by moving the molecule along a cone. The angle 240 of this cone is hereinafter referred to as a cone angle. Then, by detecting the variation of the average molecule axis direction with two polarizers, the liquid crystal element can be utilized as an electric optical element.

The electric optical element has an efficient high speed response characteristic. This is because the electric optical element utilizing the response of smectic C or H phase to electric field, has a coupling force between its spontaneous polarization and the electric field, the force being 3 or 4 orders larger than the coupling force of dielectric anisotropy compared to a TN type liquid crystal element. The electric optical element can also have a memorizing characteristic by selecting appropriate orientation control. Thus, the electric optical element can be used as a high speed optical shutter or as a display with a large information capacity.

Many kinds of chiral smectic liquid crystal materials having a ferroelectric characteristic have been studied and synthesized. The first synthesized ferroelectric liquid crystal is called DOBAMBC, this being p-decyloxybenzilidene-p'-amino-2-methylbutyl cynnamate. Many kinds of this series of liquid crystal having the following formula have been synthesized during research work into the ferroelectric liquid crystal:

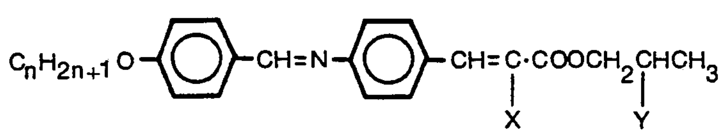

wherein X is —H, —Cl, —CN, and Y is —Cl—. This series of liquid crystals shows the chiral smectic phase at a temperature higher than room temperature. Thus, there are such defects as it not being able to be used at room temperature, it is Shiff's series, it is decomposed with water, and its stability is bad.

As a developed compound of this series, there is disclosed by B. I. Ostrovskii ("ferroelectric", 24, 1980, 309) and by A. Hallsby (Molecular crystals and liquid crystals, Letter 82, 1982, 61), a Shiff's series chiral smectic liquid crystal compound in which an introduced hydroxyl group is brought into the benzene ring, and has a hydrogen bond in the molecule. This developed compound is a compound showing smectic C* phase over a wide range of temperature including room temperature. The developed compound has the following formula

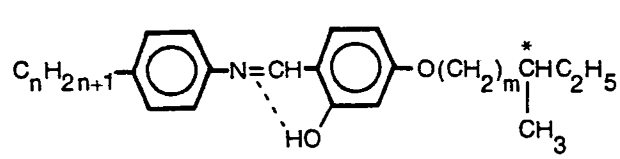

Since the developed compound also has a hydrogen bond within the molecule, it is hardly decomposed by water, and it has a much better stability as compared to a general Shiff's series liquid

crystal. As a practical matter, it is required not to crystalize at a temperature below 0°C, and so it is still not enough with only the liquid crystal material synthesized with the compound of this series.

A liquid crystal material of the azoxy series was disclosed by P. Keller et al in Annales de physique, 1978, 139. However, this compound also gives practical problems, for example by not having an efficient charateristic concerning the temperature range, and by being a deep yellow in colour.

Among these compounds, an ester series liquid crystal having a good stability and widely practiced as a TN type liquid crystal material is given much attention, the liquid crystal compound having the formula:

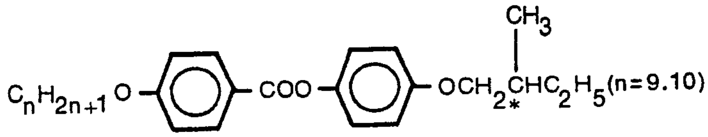

The liquid crystal compound having the above formula was disclosed by B. I. Ostrovskii et al as being material shwoing a chiral smectic liquid crystal at a temperature range close to room temperature. Also, G. W. Gray et al disclosed in "Molecular crystal and liquid crystal" 37 (1976) 189, (1978) 37, a biphenyl ester series material showing a chiral smectic liquid crystal at a high temperature range.

As stated above, at the present time, a liquid crystal that shows a chiral smectic phase over a wide range of temperatures including the practical condition of room temperature, does not exist. Furthermore, even with materials showing a chiral smectic phase over a relatively wide range of temperatures, there are still some stability problems.

Therefore, the present invention is directed towards providing a new liquid crystal compound which has good stability, and which is highly likely to give a chiral smectic liquid crystal compound over a wide range of temperatures including room temperature.

Accordingly, the present invention provides a ferroelectric smectic liquid crystal compound having the general formula:

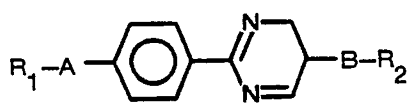

wherein:

A and B respectively are —, —O—,

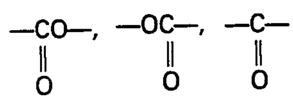

(— is a direct bond);

one of $R_1$ and $R_2$ is a straight chain alkyl group, and the other is either an alkyl group having an asymmetric carbon atom or an alkoxyl alkyl group having an asymmetric carbon atom and ether bonding; and

onto the asymmetric carbon atom there is added one of the groups $—CH_3$, —CN, —Cl.

Many of the liquid crystal compounds shown in the above formula show Sm C* phase over a wide temperature range including room temperature, and their response speed is very fast. In this connection the respective speed of this series of liquid crystal compounds is faster than the compounds shown in the general formula:

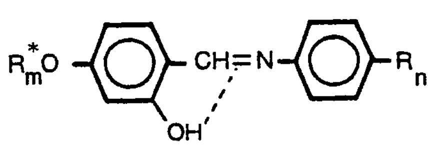 or 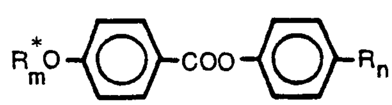

wherein:

$R_m$* is a chiral alkyl chain having an asymmetric carbon atom in it, and

$R_n$ is a straight chain alkyl group.

It is thought that, although the framework portion of the molecule is short as the benzene ring and the pyrimidine ring are directly bonded, the fact that the pyrimidine series liquid crystal compound of the present invention shows a high smectic characteristic, and that it shows the Sm C* phase over a wide temperature range, is because the polarization of the molecule axis direction, caused by the difference between the electric negativity of the pyrimidine ring and the benzene ring, and mutual function between the molecules, are large. Also, the high-response ability may be due to the fact that, as the width of the pyrimidine ring is wider than the width of the benzene ring, the molecule form is inflated in the center portion. This inflated part may control the distance between the molecules. Thus, the rotation viscosity of the molecule is small and the speed of the response becomes faster.

The ferro-electric smectic liquid crystal compound may have the formula

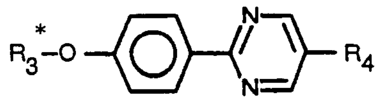

wherein $R_3$* is an alkyl group that has an asymmetric carbon atom and $R_4$ is a straight chain alkyl group.

The ferro-electric smectic liquid crystal compound may have the formula

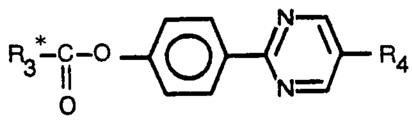

wherein $R_3$* is an alkyl group having an asymmetric carbon atom and $R_4$ is a straight chain alkyl group.

The ferro-electric smectic liquid crystal compound may have the formula

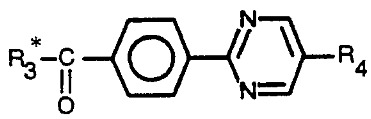

wherein $R_3$* is an alkyl group having an asymmetric carbon atom and $R_4$ is a straight alkyl group.

A ferro-electric smectic liquid crystal compound may have the formula

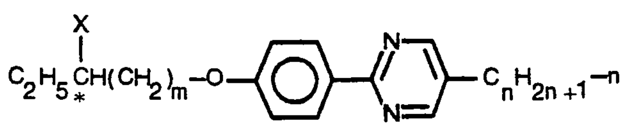

wherein m is an integral number from 0 to 8, n is an integral number from 5 to 14, X is either —Cl or —CN, * is an aysmmetric carbon atom, and the alkyl group containing —n being a straight chain.

A ferro-electric smectic liquid crystal compound may have the formula

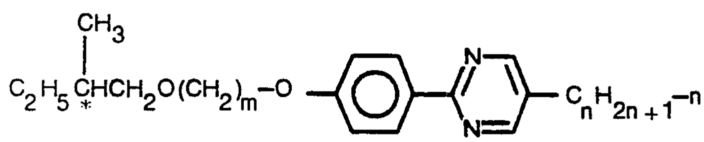

wherein m is an integral number from 1 to 8, n is an integral number from 5 to 14, * is an aysmmetric carbon atom, and the alkyl group containing —n being a straight chain.

The ferro-electric smectic liquid crystal compound may have the formula

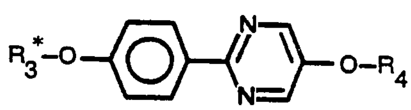

where $R_3$* is an alkyl group having an asymmetric carbon atom, and $R_4$ is a straight chain alkyl group.

The ferro-electric smectic liquid crystal compound may have the formula

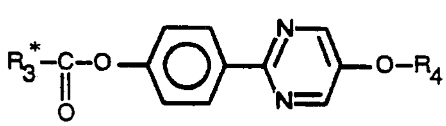

wherein $R_3$* is an alkyl group having an asymmetric carbon atom, and $R_4$ is a straight chain alkyl group.

The ferro-electric smectic liquid crystal compound may have the formula

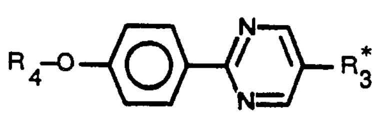

wherein $R_3$* is an alkyl group having an asymmetric carbon atom, and $R_4$ is a straight chain alkyl group.

The ferro-electric smectic liquid crystal compound may have the formula

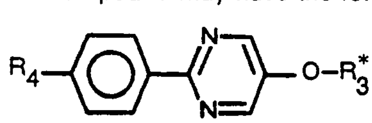

wherein $R_3$* is an alkyl group having an asymmetric carbon atom and $R_4$ is a straight chain alkyl group.

A ferro-electric smectic liquid crystal compound may have the formula

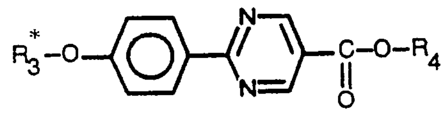

wherein $R_3$* is an alkyl group having an asymmetric carbon atom and $R_4$ is a straight chain alkyl group.

A ferro-electric smectic liquid crystal compound may have the formula

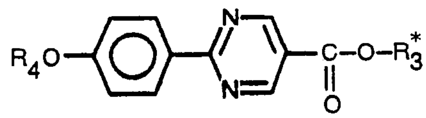

wherein $R_3$* is an alkyl group having an asymmetric carbon atom, and $R_4$ is a straight chain alkyl group.

The ferro-electric smectic liquid crystal compound may have the formula

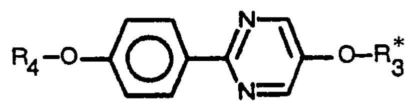

wherein $R_3$* is an alkyl group having an asymmetric carbon atom, and $R_4$ is a straight chain alkyl group.

A ferro-electric smectic liquid crystal compound may have the formula

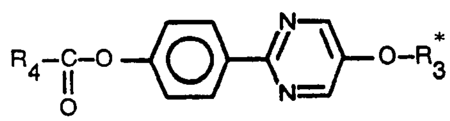

wherein $R_3$* is an alkyl group having an asymmetric carbon atom, and $R_4$ is a straight chain alkyl group.

According to the present invention, there is also provided an additive compound for a ferro-electric smectic liquid crystal mixture having the general formula

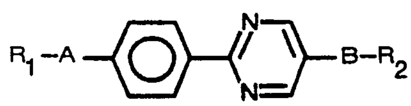

wherein A and B respectively are —, —O—,

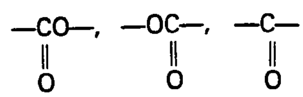

(— is a direct bond);

one of $R_1$ and $R_2$ is a straight chain alkyl group, and the other is either an alkyl group having an asymmetric carbon atom or an alkoxy alkyl group having an asymmetric carbon atom and ether bonding; and

onto the asymmetric carbon atom there is added one of the groups $—CH_3$, —CN, Cl.

The additive compound for the ferro-electric smectic liquid crystal mixture may have the formula

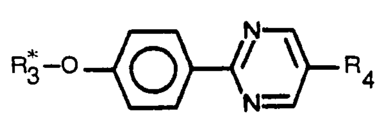

wherein $R_3$* is an alkyl group that has an asymmetric carbon atom, and $R_4$ is a straight chain alkyl group.

The additive compound for the ferro-electric smectic liquid crystal mixture may have the formula

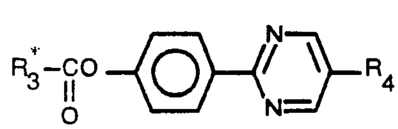

wherein $R_3$* is an alkyl group having an asymmetric carbon atom, and $R_4$ is a straight chain alkyl group.

The additive compound for the ferro-electric smectic liquid crystal mixture may have the formula

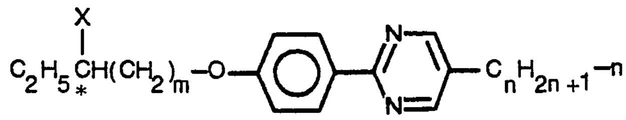

wherein m is an integral number from 0 to 8, n is an integral number from 5 to 14, X is either —Cl or —CN, * is an asymmetric carbon atom, and the alkyl group containing —n being a straight chain.

The additive compound for the ferro-electric smectic liquid crystal mixture may have the formula:

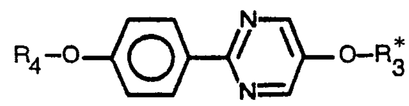

wherein $R_3$* is an alkyl group having an asymmetric carbon atom, and $R_4$ is a straight chain alkyl group.

The additive compound for the ferro-electric smectic liquid crystal mixture may have the formula

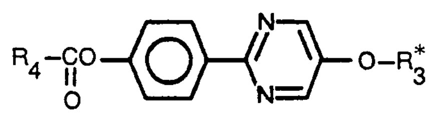

wherein $R_3$* is an alkyl group having an asymmetric carbon atom and $R_4$ is a straight chain alkyl group.

In order to facilitate a complete and easy understanding of the present invention, reference will now be made to the accompanying drawings and to the following Examples.

In the drawings:

Figure 1 is a typical diagram of smectic C* phase or smectic H phase;

Figure 2 is a typical drawing showing the movement of a chiral smectic phase liquid crystal molecule moving along a cone by an electric field;

Figure 3 is a graph showing the temperature dependance of the response speed; and

Figures 4 to 8 are graphs showing the variation of response speed when n is fixed at 8—12 and m is varied.

Figures 1 and 2 will be understood from the above described disadvantages of the prior art.

The liquid crystal compounds of the present invention may be synthesized as follows:

Synthetic method 1

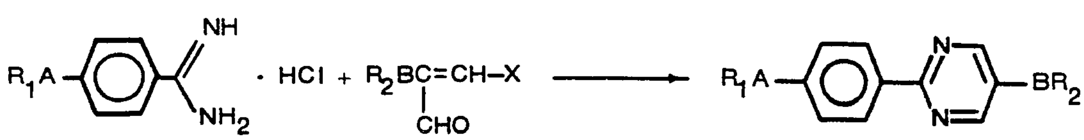

wherein X is a lower alkyl diamine group, a hydroxyl group, or a lower alkoxy group, and A and B are as defined above.

The reaction is effected by selecting an appropriate solvent, and by utilizing an alkali metal alcoholate such as alkali metal sodium alcoholate, potassium-t-butoxyde, and an anhydrous alkali metal salt such as anhydrous sodium carbonate or anhydrous potassium carbonate. The following solvents are examples of appropriate solvents: alcohols such as methanol, ethanol, propanol, isopropanol, or butanol; and glycols such as benzene, toluene, dimethyl formamide, dimethyl sulfoxide, tetrahydrofuran, ethylene glycol dimethyl ether, or diethylene glycol monomethyl ether. The reaction temperature may be room temperature, or the reflux temperature of the solvent.

Synthetic method 2

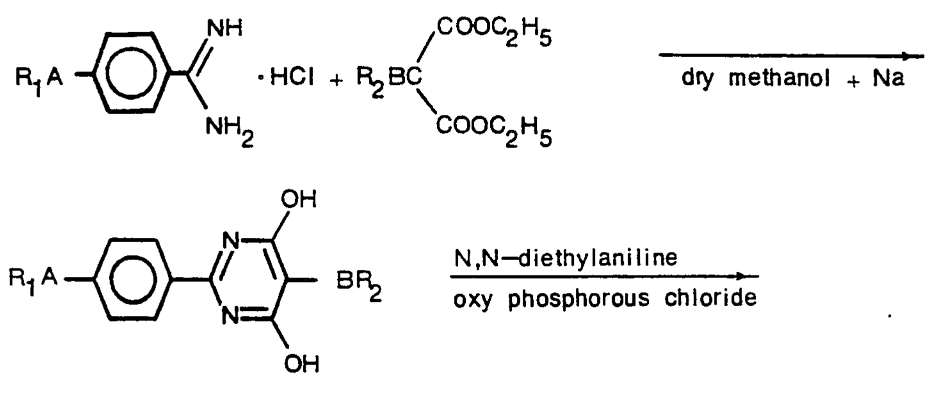

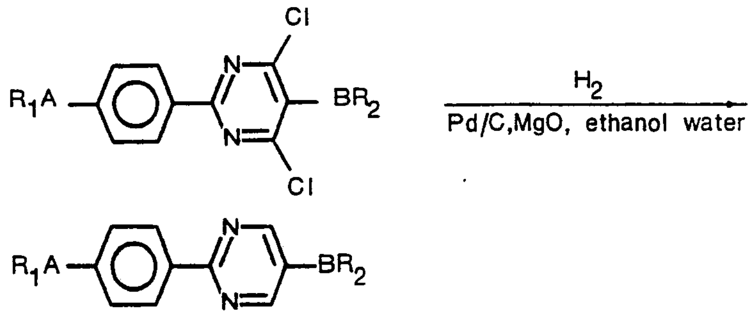


where $R_1$, A, B, and $R_2$ are as defined above.

The reaction of the first formula occurs when alkali metal alcoholate and anhydrous alkali metal salt are brought together. In the reaction of the second formula, thionyl chloride, phosphorous trichloride, or phosphorous penta chloride may be used. Phosphorous trichloride can be utilized other than oxy phosphorous chloride, N,N-diethylaniline can be replaced with a tertiary amine such as pyridine or triethylamine. The third reaction formula shows the de-halogenation by a contact reduction reaction.

Here,

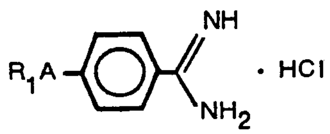

is synthesized as follows:

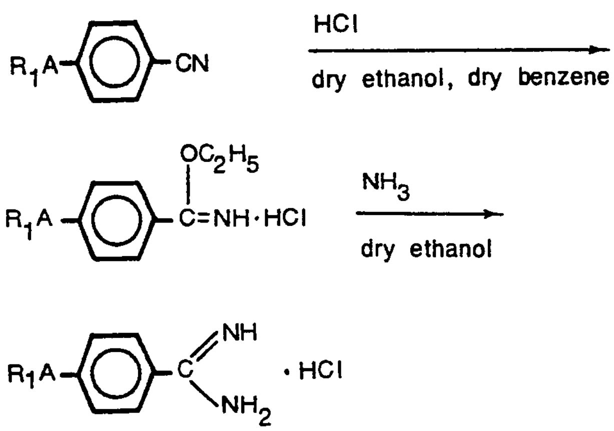


Synthetic method 3

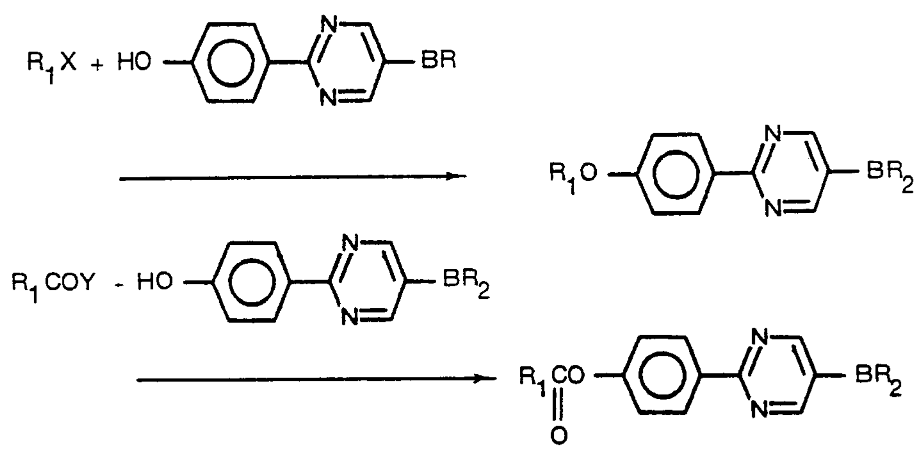

wherein X in the formula (a) is a tolyl sulfonyloxy group, a methansulfonyloxy group, or a halogen atom; Y in the formula (b) is a halogen atom or an active ester; and $R_1$, B and $R_2$ are as defined above.

The reaction of formula (a) is conducted with an appropriate solvent such as dimethyl formamide, dimethyl sulfoxide, toluene, xylene or dioxane. The condensation reaction is effected utilizing an alkali metal or an alkali metal hydride.

In the reaction of formula (b), the solvent may be a solvent of the ether series such as ethyl ether or tetra hydrofuran, droxan, a solvent of aromatic series such as benzene, toluene, a solvent of the ester series such as ethyl ester acetate, or butyl ester acetate, or a solvent of the haloalkane series such as chloroform. In order to effect a smooth reaction, a catalyst such as tertiary amine, for example pyridine, triethylamine, dimethylaniline or dimethylamino pyridine, may be used.

Here

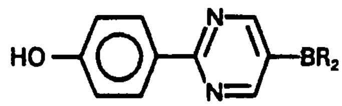

can be synthesized as follows:

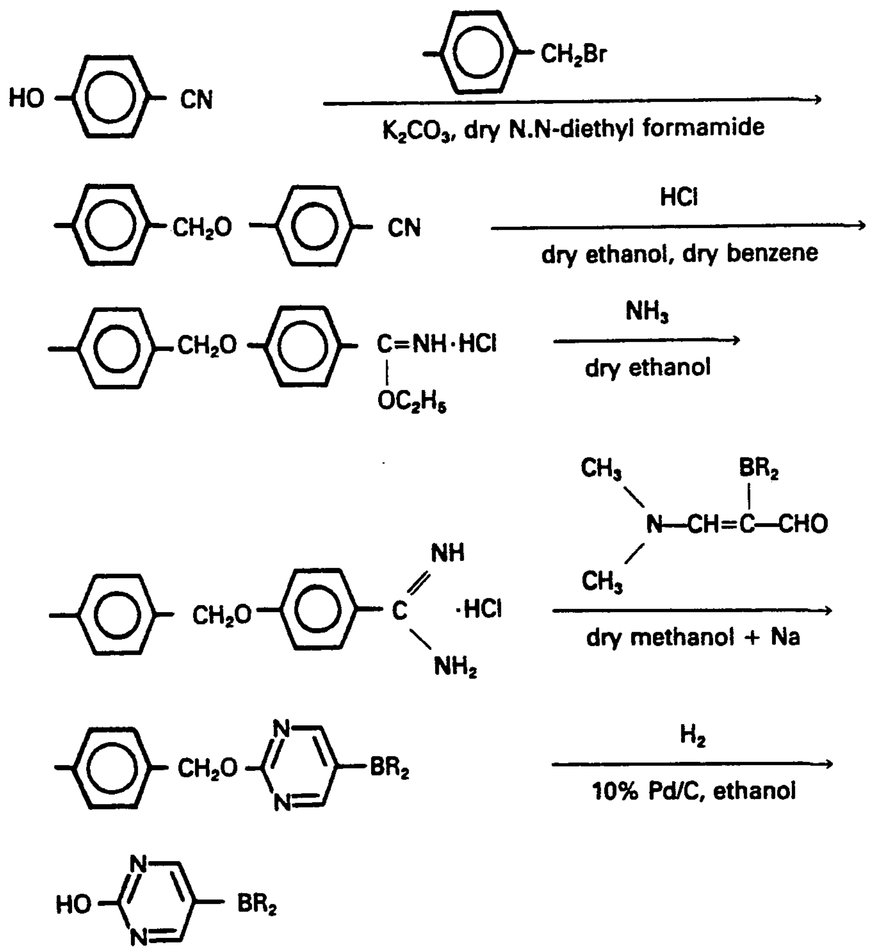


Methods for synthesizing phenyl pyrimidine having various side chains are stated above. There will now be given methods of synthesizing optically active side chains.

Thus, optically active alcohol may be synthesized as follows:

Utilizing neat active amyl alcohol of the formula

$$\begin{array}{c} CH_3 \\ | \\ CH_3CH_2\overset{}{\underset{*}{C}}HCH_2OH, \ [\alpha]_D^{20} = -4.487 \end{array}$$

as the starting material, a reaction is effected to increase carbon using an alkyl malonic acid synthetic method. Then there is obtained the following compound which has an increased number of carbon atoms.

$$\begin{array}{c} CH_3 \\ | \\ CH_3CH_2\underset{*}{C}H(CH_2)_m\text{—}OH \end{array}$$

The optically active alcohol thus made, can be changed to an aliphatic or aromatic compound, sulfonic acid ester, or halogenide, by usual methods.

With this method, the asymmetric carbon atom becomes the third carbon atom counting from the edge of the side chain.

The method of synthesizing a generally optically active side chain in which an asymmetric carbon atom is positioned in one other place, is as follows:

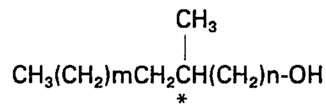

(1)

wherein m + n are an integral number from 1 to 16, m = 0 to 13, n = 1 to 14 and * is an asymmetric carbon atom.

To obtain the chemical compound shown in formula (1), a Grignard reagent having the following formula

$CH_3(CH_2)mMgX$ (2)

wherein X is a halogen atom, may be contacted with the following compounds (a), (b) and (c):

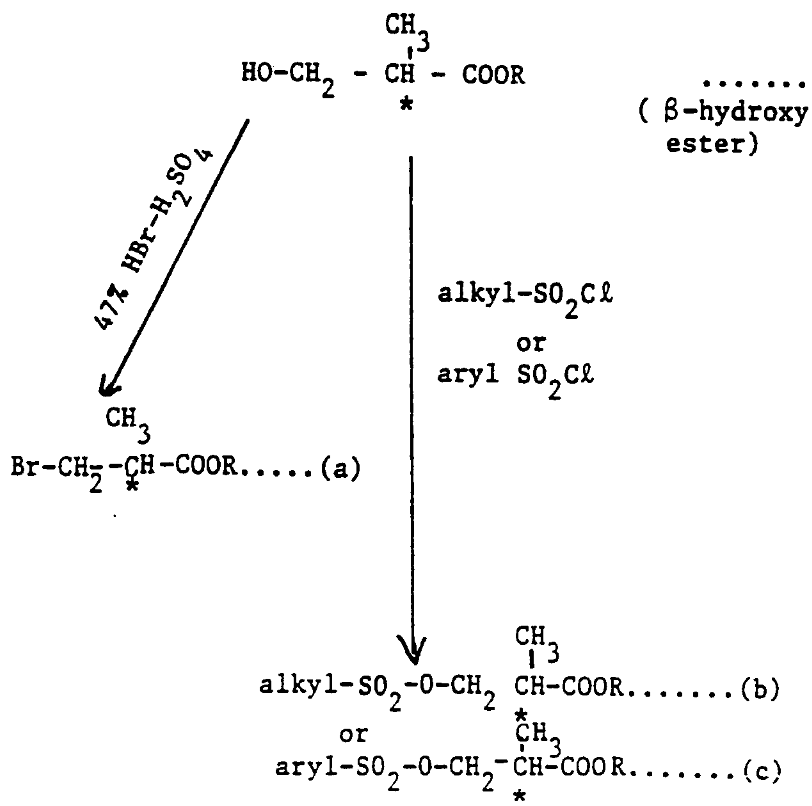


..............(3)
( β-hydroxy isobutyric acid ester)

.....(a)

.......(b)

or

.......(c)

wherein R is as defined below, and * is an asymmetric carbon atom.

Next, conduct a coupling reaction onto the compound shown in formula (2) and the compound shown in formula (a), (b) and (c) using a catalyst such as a halide metal (for example, primary copper iodide, primary copper bromide, or secondary iron chloride) and then obtain the compound as shown in formula (4)

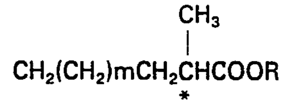

(4)

wherein m is an integral number 0 to 13, R is a lower alkyl group such as methyl, ether, propyl, and * is an asymmetric carbon atom.

The compound obtained in the reaction shown in formula (4) is changed into the alcohol compound shown in formula (1), by being reduced with lithium aluminium hydride.

In the obtained compound shown in formula (1), (wherein m is an integral number from 0 to 13, n is 1, and * is an asymmetric carbon atom), the hydroxy group is changed into a halogen, a methane sulfonyl oxy group, or p-toluene sulfonyl oxy group, by a usual halogenide reaction or by a sulfonyl esterification reaction.

The compounds obtained with the above methods are converted to a compound with a differently positioned asymmetric carbon atom, by appropriately selecting and conducting a usual increasing carbon reaction (for example) using the method to conduct a reduction after malonic ester synthetic method, the method to conduct a reduction, hydrolysis, after cyano-ized, or the method to conduct a reduction to form carboxylic acid after Grignard reaction).

There is thus obtained the optically active alkanol compound shown in formula (1) having one asymmetric carbon atom at an arbitrary position within the alkyl chain.

The compound shown in formula (1) is utilized as a side chain material compound, either as it is, or by halogenizing or sulfonyl esterizing the hydroxyl group.

Because the liquid crystal compound of the present invention has one asymmetric carbon, there exists one pair of optical antipodes. These antipodes are included in the compound of the present invention. The optical characteristic is determined in the compound shown in formula (1), by selecting which antipode to use.

Specific reference will now be made to the following Examples.

## Example 1
Synethesizing optically active (s)-5-n-octyl-2-[4-(6-methyloctyloxy)phenyl]pyrimidine.

1) Synthesizing optically active (s)-4-(6-methyl octyl oxy) benzonitrile.

Pour in 5.75 g 4-cyano phenol, 10 g of 1 brom-6-methyl octane synthesized of available active amyl alcohol, 6.67 g of anhydrous potassium carbonate, and 30 ml of N,N-dimethyl formamide into a 100 ml four-mouth flask, under a nitrogen atmosphere, and conduct a reaction for 8 hours at 110°C. After the reaction, filter the insoluble matter and then extract ether. The organic layer is then washed with 5% NaOH, water and saturated salt water, and then dried. Then evaporate and remove the ether. Then, when the obtained oily matter is refined, 11.4 g of optical (s)-4-(6-methyloctyloxy)benzonitrile are obtained having the following characteristics

$\nu_{max}^{Film}(cm^{-1})$: 2230, 1605, 1570, 1115
$\delta_{TMS}^{CDCl_3}$(ppm): 6.92, dJ=9Hz:
2H, Aromatic H
7.57, dJ=9Hz, 2H, Aromatic H
3.97, tJ=6Hz; 2H, $—CH_2—O—$

2) Synthesizing optically active (s)-4-(6-methyloctyl oxy) benzamidine hydrochloric acid salt.

Pour 10 g of optically active (s)-4-(6-methyloctyloxy) benzonitrile, 12.5 ml dry ethanol, and 16 ml dry benzene into a 100 ml four-mouth flask. Agitate this mixture. Agitate dried hydrogen chloride gas at a temperature below 3°C, and the blow it in at below 3°C. Then leave at room temperature for 2 days, evaporate and remove the solvent, to obtain an impure crystal.

Put the obtained impure crystal into a 300 ml four-mouth flask with 64 ml of dry ethanol, and then slowly drip 30 ml of dry ethanol which includes 11.08 g ammonia, at room temperature. After dripping, the mixture is left at room temperature for three days and is then refined to obtain 9.58 g of optical (s)-4-(6-methyloctyloxy) benzamidine hydrochloric acid salt having the following characteristics.

$\nu_{max}^{nujol}(cm^{-1})$: 3060, 1680, 1655, 1105
DMSO-$d_6$ $\delta_{TMS}$(ppm) 9.4, broads, 3H,

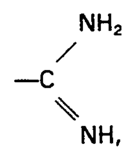

(D$_2$O exchange characteristic):
7.14, d J=9Hz, 2H, Aromatic H
8.06, d J=9Hz, 2H, Aromatic H
4.09, t J=6Hz, 2H, $—O—CH_2—$

3) Synthesizing optically active (s)-5-n-octyl-2-[4-(6-methyl octyl oxy)phenyl]-4,6-dihydroxy pyrimidine.

Pour 1.15 g metal sodium and 33 ml dry methanol into a 100 ml four-mouth flask. Pour 5 g of optically active (s)-4-(6-methyloctyloxy) benzamidine hydrochloric acid salt, and then pour 4.55 g n-octylmalonic acid diethyl into this methylate sodium solution. Then conduct a reaction for 18 hours under a heat reflux condition. After cooling acidify the products of the reaction utilizing concentrated sulfuric acid, and then extract the impure crystal which is obtained.

The impure crystal is refined, and then optically active 6.32 g of (s)-5-n-octyl-2-[4-(6-methyloctyloxy)-phenyl]-4,6-dihydroxy-pyramidine are obtained having the following characteristics.

$\nu_{max}^{nujol}(cm^{-1})$: 2660, 1664, 1610, 1110

4) Synthesizing optically active (s)-5-n-octyl-2[4-(6-methyloctyl oxy)phenyl]-4,6-dichloro-pyrimidine.

Pour 6 g of optically active (s)-5-n-octyl-2-[4-(6-methyloctyloxy)phenyl]-4,6-dihydroxy-pyrimidine, 27 ml of oxy phosphorous chloride, and 4 ml of N-diethyl aniline into a 50 ml flasco, and then conduct a reaction for 21 hours under heat reflux conditions. After the reaction, evaporate and remove the obtained oxy phosophorous chloride, and then pour into ice water. Then extract from this, wash with alkali water solution, then wash again with water and saturated salt water until it is neutral. After drying the organic layer, evaporate and remove the ether to obtain an impure product. When this product is refined, 3.6 g of optically active (s)-5-n-octyl-2-[4-(6-methyl octyl oxy)phenyl]-4,6-dichloro-pyrimidine are obtained having the following characteristics.

$\nu_{max}^{nujol}(cm^{-1})$: 1610, 1122, 1090

$\delta_{TMS}^{CDCl_3}(ppm)$:

8.22, d J=9Hz, 2H, Aromatic H

6.85, d J=9Hz, 2H, Aromatic H

3.94, t J=9Hz, 2H, $-CH_2-O-$

2.82, t, 2H, $-CH_2$ 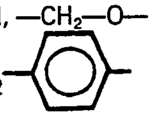

5) Synthesizing optically active (s)-5-n-octyl-2-[4-(6-methyl octyl oxy)phenyl]-pyrimidine.

Pour 1.88 g of optically active (s)-5-n-octyl-2-[4-(6-methyloctyloxy)phenyl]-4,6-dichloro-pyrimidine, 0.4 g of 10% palladium-carbon, 0.55 g of magnesium oxide, 60 ml of ethanol, and 45 ml of water, into a 200 ml flask. Then add hydrogen until an appropriate amount of the hydrogen at oil bath condition at 50°C, is absorbed. The catalyst is filtered and separated, and then ether is extracted. The ether layer is then washed with water and with saturated salt water, and then dried. The ether is evaporated and removed. The obtained impure product is repeatedly refined, and 0.9 g of optically active (s)-5-n-octyl-2-[4-(6-methyloctyloxy)phenyl]-pyrimidine are obtained having the following characteristics.

$\nu_{max}^{nujol}(cm^{-1})$: 1610, 1584, 1110

$\delta_{TMS}^{CDCl_3}(ppm)$:

8.55, S, 2H 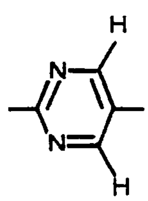

8.35, d J=9Hz, 2H, Aromatic

6.94, d J=9Hz, 2H, Aromatic

3.99, t J=6Hz, 2H, $-CH_2-O-$

2.57, t, 2H, $-CH_2$ 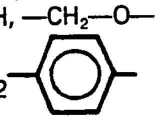

This liquid crystal compound has a transmitting temperature as follows:

$$Cry \underset{3°C}{\overset{3°C}{\rightleftarrows}} Sm\ B \underset{*}{\overset{14.2°C}{\rightleftarrows}} Sm\ C^* \xrightarrow{48.6°C} Sm\ A \xrightarrow{56.3°C} Iso$$

(* stands for super-cooling).

To show Sm C* phase at a wide temperature range including room temperature for a range of about 35 degrees (°C), and also to have SM B phase under the Sm C* phase, the smectic domain condition is kept at 3°C at the low temperature side. The Sm B phase does not respond to display. Because there is no destruction of the smectic domain condition by the crystalization, when at high temperature and back to Sm C* phase, the same display condition is turned ON/OFF. Thus Sm B phase may be utilized for the preservation under low temperature of the LC panel time.

This liquid crystal compound is sandwiched between PVA rubbing one-axis orientation processed plates. The liquid crystal layer thickness is fixed at 2.5 µm, with ±20 V voltage applied, under orthogonal nicol. Then measure the characteristic and the result is as follows:

the measuring temperature is 25°C

the cone angle is 40.5°C

contrast (T on/T off) is 12.5

response speed is 600 µ sec.

The temperature depending data of the response of this cell is shown in Figure 3.

Examples 2—31

In a compound shown as

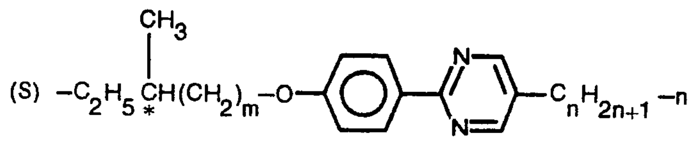

several compounds which have the above formula with m and n being respectively suitable integral numbers, are synthesized with the same method as Example 1.

The data of the transmitting temperature and the response speed measured with the same method as in Example 1, is shown in Table 1.

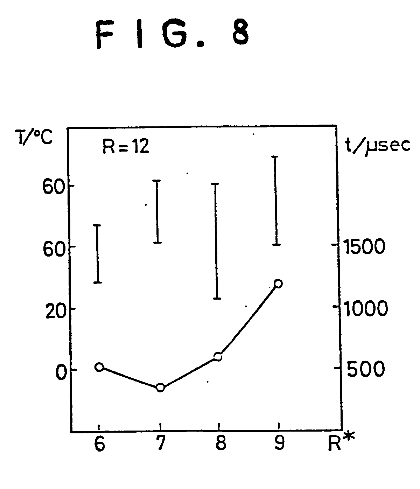

Figures 4 and 8 show the Sm C* temperature range and response speed of the above compound of Table 1, drawn in relation to the carbon number of the side chain.

TABLE 1

| Example No. | m | n | phase transition temperature | response speed µsec |
|---|---|---|---|---|
| 2 | 1 | 11 | Cry ⇄ Sm C* (39.9 →) ; Sm C* → Sm A (41.4) ; Sm A → Iso (47.5) | 260 (28.3°C) |
| 3 | 2 | 6 | Cry ⇄ Ch (13 →, *8 ←) ; Ch ⇄ Iso (32 →) | — |
| 4 | 2 | 7 | Cry → Iso (39.2) ; Iso → Sm A (39.2) ; Sm A → Cry (*10) | — |
| 5 | 2 | 8 | Cry → Iso (36.4) ; Iso → Sm A (36.4) ; Sm A → Cry (*28) | — |
| 6 | 2 | 9 | Cry ⇄ Sm X (23.0 →) ; Sm X ⇄ Sm C (31.0 →) ; Sm C → Sm A (*38.7) ; Sm A → Iso (46.5) | 330 (30°C) |
| 7 | 2 | 10 | Cry ⇄ Sm C (22 →, *5.8 ←) ; Sm C → Sm A (*41.4) ; Sm A → Iso (41.5) | 610 (33°C) |
| 8 | 2 | 11 | Cry ⇄ Sm X (24.7 →, 18.2 ←) ; Sm X ⇄ Sm C (33.9 →, * ←) ; Sm C → Sm A (*45.5) ; Sm A → Iso (50.0) | 530 (39°C) |
| 9 | 2 | 12 | Cry ⇄ Sm C (28.5 →, *18.7 ←) ; Sm C → Sm A (*47.0) ; Sm A → Iso (51.2) | 540 (34°C) |
| 10 | 3 | 6 | Cry → Ch (25.0) ; Ch → Iso (42.5) ; Ch → Sm X (11.6) ; Sm X → Cry (*8.6) | — |
| 11 | 3 | 7 | Cry ⇄ Sm C (28.5 →, *13.0 ←) ; Sm C → Sm A (*53.6) ; Sm A → Iso (60.0) | 290 |
| 12 | 3 | 8 | Cry → Sm C (31.2) ; Sm C → Sm A (*46.8) ; Sm A → Iso (50.8) ; Sm C → Sm B (*16.8°C) ; Sm B → Cry (26.2) | 250 |
| 13 | 3 | 9 | Cry ⇄ Sm X (23.0 →, *3.0 ←) ; Sm X ⇄ Sm C (28.0 →, 7.0 ←) ; Sm C → Sm A (*30.0) ; Sm A → Ch (51.5) ; Ch → Iso (52.0) | 530 |

TABLE 1 (continued)

| Example No. | m | n | phase transition temperature | response speed μsec |
|---|---|---|---|---|
| 14 | 3 | 10 | Cry ⇄ (33.0 / *12.0) Sm X —38.5→ Sm C —*58.0→ Iso | 330 |
| 15 | 3 | 11 | Cry ⇄ (35.9 / *) Sm C —*60.0→ Iso | 380 (27.4°C) |
| 16 | 3 | 12 | 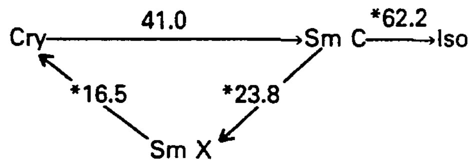 | 360 |
| 17 | 3 | 14 | Cry ⇄ (32.0 / *) Sm B ⇄ (45.0 / *29.0) Sm C —*59.8→ Iso | 450 (37.2°C) |
| 18 | 4 | 6 | Cry ⇄ (−5°C / *2) Sm A —27→ Ch —42→ Iso | — |
| 19 | 4 | 7 | Cry ⇄ (−6 / *−11) Sm A —46.3→ Ch —49→ Iso | — |
| 20 | 4 | 8 | Cry ⇄ (12.0 / *6.3) Sm C —*34.7→ Sm A —49.5→ Iso | 260 (30°C) |
| 21 | 4 | 9 | Cry ⇄ (10 / *5) Sm C —*46.0→ Sm A —59.0→ Iso | 430 (35°C) |
| 22 | 4 | 10 | Cry ⇄ (17 / *11) Sm C —*53.8→ Sm A —63.0→ Iso | 600 (30°C) |
| 23 | 4 | 11 | Cry ⇄ (20 / *8) Sm C —*59.0→ Iso | 530 (30°C) |
| 24 | 4 | 12 | 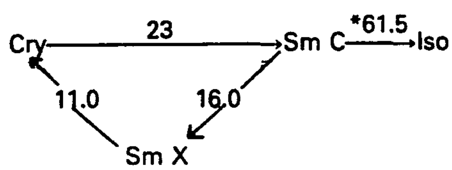 | 620 (27°C) |
| 25 | 5 | 6 | Cry ⇄ (12.0 / *8.0) Sm C —*23.8→ Ch —45.6→ Iso | 810 (17°C) |

TABLE 1 (continued)

| Example No. | m | n | phase transition temperature | response speed μsec |
|---|---|---|---|---|
| 26 | 5 | 7 | Cry $\xrightleftharpoons{10}$ Sm X $\xrightleftharpoons[*-2]{16}$ Sm C $\xrightarrow{*39.0}$ Sm A $\xrightarrow{54.0}$ Ch $\xrightarrow{61.0}$ Iso | 250 (31°C) |
| 27 | 5 | 9 | Cry $\xrightleftharpoons[*10.0]{16.0}$ Sm C $\xrightarrow{*49.1}$ Sm A $\xrightarrow{61.0}$ Iso | 700 (31°C) |
| 28 | 5 | 10 | Cry $\xrightarrow{41.0}$ Sm C $\xrightarrow{*61.0}$ Iso; Sm C $\xrightarrow{*}$ Sm X $\xrightarrow{30.0}$ Cry | 1600 (42°C) |
| 29 | 5 | 11 | Cry $\longrightarrow$ Sm B $\xrightleftharpoons[*23.0]{36.7}$ Sm C $\xrightarrow{*68.0}$ Iso | 1100 (31.5°C) |
| 30 | 5 | 12 | Cry $\xrightleftharpoons[29.3]{40.5}$ Sm C $\xrightarrow{*70.0}$ Iso | 1200 (45°C) |
| 31 | 5 | 14 | Cry $\xrightleftharpoons[*]{43.0}$ Sm B $\xrightleftharpoons[*35.5]{45.0}$ Sm C $\xrightarrow{*66.0}$ Iso | 1700 (66°C) |

Example 32

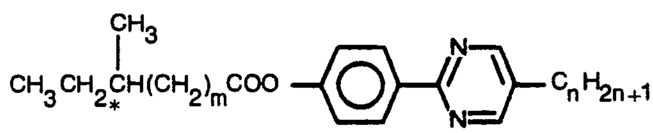

(m = 4, n = 8, * = asymmetric carbon).

A method of synthesizing optically active (s)-5-octyl-2-[4-(6-methyloctanoyloxy)phenyl]pyrimidine is as follows.

Pour 1.78 g (62 mmol) 4-(5-octyl-2-pyrimidine)phenol, and 15 ml dry pyrimidine into a 50 ml flask. Next, slowly drip 1.10 g (6.2 mmol) of optically active (s)-6-methyl octyl acid chloride under an ice cooling condition. After dripping, conduct the reaction for one whole day, returning the temperature to room temperature. After the reaction, pour the mixture into ice water, and then extract the product with ethyl acetate. The organic layer is successively washed with water, 2NHCl, 5% NaOH, then washed again with water and saturated salt water until it becomes neutral. The organic layer is then dried and the solvent is filtered and removed. The obtained impure product is repeatedly refined, and 5-n-octyl-2-[4-(6-methyloctanoyloxy)phenyl]pyrimidine 1.5 g (57%) is obtained having the following characteristics.

IR (nujol): $\nu = 1762.1588\ cm^{-1}$

$^1$H-NMR (60MHz, $CDCl_3$/TMSint) φ (ppm) =

8.62 (S, 2H, Pyrimidine H);

8.48 (d, 2H, J=9Hz, Aromatic H);

7.12 (d, 2H, J=9Hz, Aromatic H)

2.38 ~ 2.80 (m, 4H, $-CH_2-\overset{O}{\overset{\|}{C}}$, $-CH_2$-Pyrimidine

The transform temperature of this liquid crystal compound is as follows.

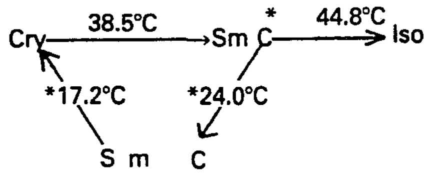


(* stands for super-cooling)

This liquid crystal compound was sandwiched between PVA rubbing one-axis orientation processed plates, the liquid crystal layer thickness was fixed at 2.5 μm, and ±20 V voltage was applied. The characteristic under orthogonal nicol condition was then measured. The measuring temperature was 35°C.

| | |
|---|---|
| Contrast (T on/T off) | 11.5 |
| Response speed | 3ms |

Examples 33—38

An object compound with a similar operation to Example 1 was obtained utilizing the below mentioned optically active alkyl acid halide and alkyl pyrimidine phenol.

TABLE 2

| starting material | | | | | object compound | | |
|---|---|---|---|---|---|---|---|
| m | $CH_3$<br>\|<br>$CH_3CH_2\underset{*}{C}H(CH_2)_mCOCl$ | n | 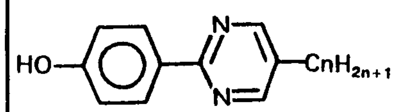 | yield (%) | $CH_3$<br>\|<br>$CH_3CH_2\underset{*}{C}H(CH_2)m\text{-}COO$— 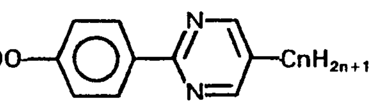 (* asymmetric carbon) | | |
| | | | | | m<br>n | I.R. (nujol) $\nu(cm^{-1})$ | H-N.M.R. ($COCl_3$/TMSint) δ (ppm) |
| 2 | $CH_3$<br>\|<br>$CH_3CH_2\underset{*}{C}H(CH_2)_2COCl$ | 8 | 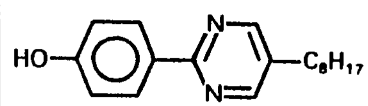 | 69 | m = 2<br>n = 8 | 1760<br>1585 | 8.56 (S, 2H, Pyrimidine H)<br>8.45 (d, 2H, Aromatic H)<br>7.18 (d, 2H, Aromatic H)<br>2.37 ~ 2.88<br>(m, 4H, —$CH_2$—CO—, —$CH_2$—Pyrimidine) |
| 0 | $CH_3$<br>\|<br>$CH_3CH_2\underset{*}{C}HCOCl$ | 11 | 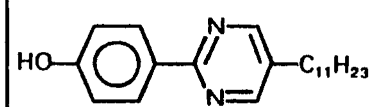 | 67 | m = 0<br>n = 11 | 1758<br>1585 | 8.58 (S, 2H, Pyrimidine H)<br>8.45 (d, 2H, Aromatic H)<br>7.16 (d, 2H, Aromatic H)<br>2.5 ~ 2.90<br>(m, 3H, —CH($CH_3$)—CO, —$CH_2$—Pyrimidine)<br>1.29 (d, 3H, CH($CH_3$)—CO)<br>1.04 (t, 3H, —$CH_2$—$CH_3$) |
| 2 | $CH_3$<br>\|<br>$CH_3CH_2\underset{*}{C}H(CH_2)_2COCl$ | 11 | 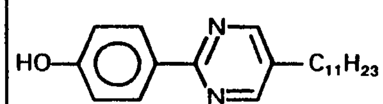 | 65 | m = 2<br>n = 11 | 1760<br>1585 | 8.56 (S, 2H, Pyrimidine H)<br>8.42 (d, 2H, Aromatic H)<br>7.16 (d, 2H, Aromatic H)<br>2.40 ~ 2.85<br>(m, 4H, —$CH_2$—CO—, —$CH_2$—Pyrimidine) |

TABLE 2 continued

| starting material | | | | | object compound | | |
|---|---|---|---|---|---|---|---|
| 4 | $CH_3$<br>\|<br>$CH_3CH_2CH(CH_2)_4COCl$<br>* | 11 | 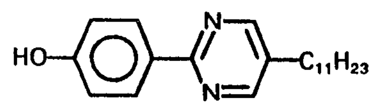 | 89 | m = 4<br>n = 11 | 1755<br>1590 | 8.62 (S, 2H, Pyrimidine H)<br>8.48 (d, 2H, Aromatic H)<br>7.22 (d, 2H, Aromatic H)<br>2.35 ~ 2.90<br>(m, 4H, —$CH_2$—CO—, —$CH_2$—Pyrimidine) |
| 2 | $CH_3$<br>\|<br>$CH_3CH_2CH(CH_2)_2COCl$<br>* | 14 | 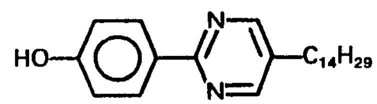 | 51 | m = 2<br>n = 14 | 1758<br>1584 | 8.57 (S, 2H, Pyrimidine H)<br>8.45 (d, 2H, Aromatic H)<br>7.17 (d, 2H, Aromatic H)<br>2.35 ~ 2.80<br>(m, 4H, —$CH_2$—CO—, —$CH_2$—Pyrimidine) |
| 4 | $CH_3CH_2CH(CH_2)_4CDCl$ | 14 | 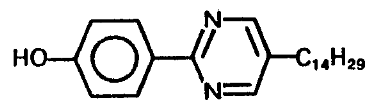 | 61 | m = 4<br>n = 14 | 1755<br>1584 | 8.57 (S, 2H, Pyrimidine H)<br>8.42 (d, 2H, Aromatic H)<br>7.12 (d, 2H, Aromatic H)<br>2.30 ~ 2.72<br>(m, 4H, —$CH_2CO$—, —$CH_2$—Pyrimidine) |

The transition temperature and the response speed of the liquid crystal, measured in a similar way to Example 1, is shown in the following table.

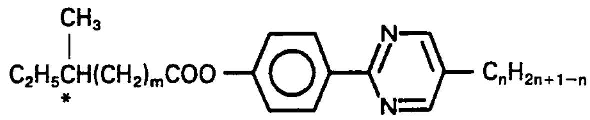

| m | n | transition temperature (°C) | response speed (measured temperature) |
|---|---|---|---|
| 2 | 8 | Cry —54.2→ Iso; Iso —*49→ Cry | — |
| 0 | 11 | Cry —52.2→ Iso; Iso —*40.7→ SmX; SmX —*→ Cry | — |
| 2 | 11 | Cry —38.1→ Sm C —*50.2→ Iso; Sm C —*29.0→ SmX; SmX —*8.2→ Cry | 28.5 µs (32.0°C) |
| 4 | 11 | Cry —62.3→ Iso; Iso —*60.0→ Sm C; Sm C —*46.5→ Sm X; Sm X —*41.2→ Cry | 350 µs (56.7°C) |
| 2 | 14 | Cry → Sm X —41.2→ Sm C —*53.1→ Iso; Sm C —*36.4→ Sm X; Sm X —*23.0→ Sm Y; Sm Y —*13.9→ Cry | 350 µs (36.7°C) |
| 4 | 14 | Cry —46.0→ Sm X —48.6→ Sm C —*62.8→ Iso; Sm C —50.0→ Sm X; Sm X —*→ Cry | 260 µs (50.2°C) |

here, Sm X, Sm Y stands for Smectic phases except for Sm A phase and Sm C* phase, so because it is not specifiable, it is noted as Sm X, Sm Y. * stands for super-cooling.

As shown in the above table, with the liquid crystal of m = 2, n = 8 and the liquid crystal m = 0, n = 11, the response speeds cannot be measured because they do not take Sm C* phase, but from various blend experiments, it has been determined that they can be utilized as a dopant to speed up the response speed.

Example 39

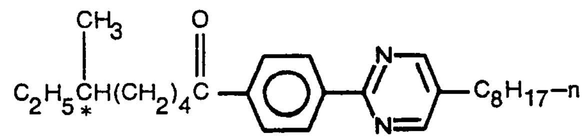

Synthesizing (s)-5-n-octyl-2-[4-(1-hydroxy-6-methyl octyl)phenyl] pyramidine.

Pour in 0.82 g of (s)-4-(1-hydroxy-6-methyloctyl benzamidine hydrochloric acid salt, and 0.56 g and β-n-octyl-α-dimethylamino acrolein. Afer resolving it in 20 ml dry ethanol, add 2.04 g 28% sodium methylate methanol solution. Conduct a reaction for 13 hours under reflux. After the reaction, pour the mixture into ice water and turn it to hydrochloric acid. Then extract the reaction product with ethyl acetate. The ethyl acetate layer is washed with water and saturated salt water, and dried. Then filter and remove the solvent. The obtained impure product is refined with silica gel chromatography and re-crystalization. Then optically active (s)-5-n-octyl-2-[4-(1-hydroxy-6-methyloctyl) phenyl] pyrimidine 0.64 g is obtained having the following characteristics.

IR ν max $cm^{-1}$: 3260, 1595, 1555, 1440, 800

$^1H$-NMR (60 MHz, $CDCl_3$) δ (ppm):

8.67 (S, 2H)
8.45 (d, 2H)
7.50 (d, 2H)
4.77 (t, 1H)
2.2 ~ 2.8 (m)

Synthesizing (s)-5-n-octyl-2-[4-(6-methyloctanoyl)phenyl]pyrimidine

Pour in 0.107 g bichromatic pyrimidinium, and 5 ml of dried N,N-dimethylformamide. Next, slowly drip 0.08 g (s)-5-n-octyl-2-[4-(1-hydroxy-6-methyloctyl)phenyl] pyrimidine dissolved in dried, N,N-dimethyl formamide 2 ml at room temperature.

When the dripping finishes, conduct a reaction for 2 hours at room temperature. After the reaction, add ether and dilute it. Then filter and separate the insoluble matters utilizing a high-flow-super-cell. Then conduct an ether extraction with the reacted product. The ether layer is washed with dilute hydrochloric acid and water, dried and filtered. The solvent is removed. Then the obtained impure product is refined with silica gel chromatography and re-crystalization, to obtain 0.028 g of (s)-5-n-octyl-2-[4-(6-methyloctanoyl)phenyl]pyrimidine having the following characteristics.

IR ν max $cm^{-1}$: 1680, 1610, 1580, 1545

$^1H$-NMR (60MHz, $CDCl_3$) δ (ppm):

0.6 ~ 2.2 (m, 30H)
2.64 (t, 2H)
3.01 (d, 2H)
8.07 (d, 2H)
8.55 (d, 2H)
8.66 (S, 2H)

The phase transition temperature of this liquid crystal compound is as follows:

$$\text{Cry} \underset{*51}{\overset{67}{\rightleftarrows}} \text{Sm}\ \overset{*}{\text{C}} \xrightarrow{.69} \text{Sm A} \xrightarrow{79.3} \text{Iso}$$

This liquid crystal compound was sandwiched between PVA rubbing one-axis orientation processed plates. The liquid crystal layer thickness was fixed at 2.5 μm, and ±20 V voltage applied. The response was measured at orthogonal nicol, and the result was that the value became 200 μs, at 61°C

Example 40

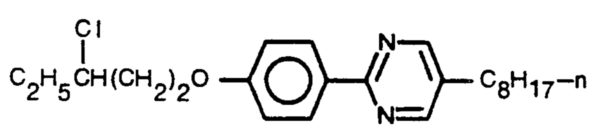

Synthesizing optically active (s)-5-n-octyl-2-[4-(3′-chloropentyloxy)phenyl]pyrimidine.

Pour 0.77 g of sodium hydride (about 50% oil suspension), and 3 ml of dry N,N-dimethyl formamide into a 10 ml two-mouth flask equipped with a dripping funnel, cooling tube and calcium chloride tube. Then drip 0.38 g of 4-(5-n-octyl-2-pyrimidinyl)phenol dissolved in 1 ml dry N,N-dimethylformamide, at a temperature below freezing point. After a reaction time of 30 minutes at room temperature, drip 0.37 g of

optically active (s)-3-chloropentyl p-toluene sulfonic acid ester (this compound was synthesized with the general method with L(+)-hydroxy valenic acid methyl ester$[[\alpha]_D^{20} = +28.1, (C=1, CHCl_3)]$ which is dissolved into 1 ml dry N,N-dimethylformamide.

When the dripping finishes, conduct a reaction for 8 hours at 100°C at an oil bath condition. After the reaction, dry the product, pour it into ice water, and extract it with ethyl acetate. The ethy acetate layer is washed with 5% NaOH and water, is dried, and then the organic solvent was filtered and removed. The remaining oil-like sediments were poured over silica gel carame chromatography. The re-crystalized crystal was repeatedly refined to obtain 0.19 g of optically active (s)-5-n-octyl-2-[4-(3'-chloropentyloxy)phenyl] pyrimidine having the following characteristics.

IR $\nu_{max}^{film}(cm^{-1})$: 1610, 1585, 1430, 1250, 1170, 1110, 800, 750

$^1$H-NMR (60MHz, $CDCl_3$/TMS int) δ (ppm):

8.62 (S, 2H, Pyrimidine H);
8.42 (d, 2H, Aromatic H);
7.02 (d, 2H, Aromatic H);
4.03 (t, 2H, $-CH_2-O-$)
2.60 (t, 2H, $-CH_2$-pyr)

The transition temperature was

$$\mathrm{Cry} \underset{*13.5}{\overset{15.0}{\rightleftarrows}} \mathrm{Sm\ A} \xrightarrow{42.6} \mathrm{Iso}$$

(here, * shows over cooling)

Here, the compound of this Example

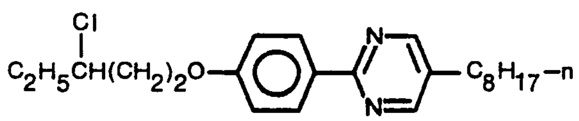

does not take the Sm C* phase, so the transition temperature was measured, and the response speed of the liquid crystal compound obtained by blending the above compound and the compound of Example 1,

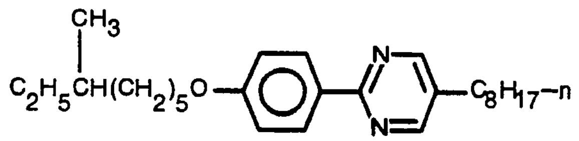

in the proportion of 1 part of the above compound of this Example per 3 parts of the above compound of Example 1.

The transition temperature was

$$\mathrm{Cry} \underset{*3°C}{\overset{10.5}{\rightleftarrows}} \mathrm{Sm\ C} \xrightarrow{*27.2} \mathrm{Sm\ A} \xrightarrow{51.7} \mathrm{Iso}$$

The response speed was 220 μs (25°C).

As is obvious from a comparison with the data of Example 1, the liquid crystal compound of this Example has the effect of speeding up the response speed.

Example 41

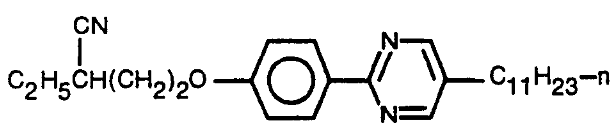

Synthesizing optically active (s)-5-n-undecyl-2-[4-(3'-cyanopentyloxy-phenyl]pyrimidine.

1) Method of synthesizing optically active (s)-5-n-undecyl-2-(4-(3'-methanesulfonyloxy-pentyloxy)phenyl]-pyrimidine.

Dissolve 2.5 g of optically active (s)-5-n-undecyl-5-[4-(3'-hydroxy-pentyloxy)phenyl]pyrimidine into 25 ml dry pyridine. Slowly drip 0.76 methane sulfonyl chloride to this compound below freezing point. After the dripping, conduct a reaction for 2 hours, then make it react for another 2 hours at room temperature. After the reaction, pour the product into ice water, and conduct ether extraction. The ether layer is washed with dilute hydrochloric acid water and water, dried, and evaporated. The ether is removed to obtain an oil-like product. This oil-like product is crystalized and separated, and 2.63 g of optically active (s)-5-n-undecyl-2-[4-(3'-methensulfonyloxy-pentyloxy)phenyl]pyrimidine are obtained having the following characteristics

IR $\nu_{max}^{nujol}$ $(cm^{-1})$: 1610, 1590, 1435, 1337, 1183, 1173, 910, 800.

2) Method of synthesizing optically active (s)-5-n-undecyl-2-[4-(3′-cyanopentyloxy)phenyl]pyrimidine

Pour 2.63 g of optically active (s)-5-n-undecyl-2-[4-(3′-methanesulfonyloxy-pentyloxy)phenyl]-pyrimidine obtained at method 1), and 10 ml of dry dimethyl sulfoxide into a 30 ml flask equipped with a cooling tube and a calcium chloride tube. After it has dissolved, add 0.32 g of sodium cyanide, and agitate it for 15 hours at 80 to 90°C. Leave it to cool down, pour it into ice water, and then conduct ether extraction to the reaction material. The ether layer is washed with dilute hydrochloric acid water and water, and dried. The ether is evaporated and removed, and an oil-like material is obtained. This oil-like material is crystallized and separated, and then 2.63 g of optically active (s)-5-n-undecyl-2-[4-(3′-cyanopentyloxy)-phenyl]pyrimidine are obtained.

The specific rotatory power is

$$[\alpha]_D^{25} = -28.57°C\ (C = 2,\ CHCl_3)$$

but the optical purity has not been fully established. Other characteristics are as follows:

IR $\nu_{max}^{nujol}$ ($cm^{-1}$): 2240, 1605, 1585, 1430, 1245, 1165, 797.

$^1$H-NMR (60 MHz, $CDCl_3$/TMS int) δ (ppm):

8.60 (S, 2H, Pyrimidine H)
8.40 (d, 2H, Aromatic H)
7.00 (d, 2H, Aromatic H)
4.20 (t, 2H, $-CH_2-O-$)
0.65 ~ 3.15 (m, 31H).

The transition temperature of the obtained compound was measured and the following result was obtained.

The transition temperature:

$$Cry \underset{*53}{\overset{60.6°C}{\rightleftarrows}} Iso$$

(wherein * shows supercooling)

Here, as the compound of this Example

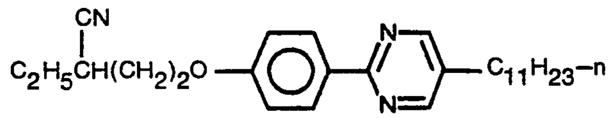

does not take the Sm C* phase, the transition temperature was measured and the response speed of the liquid crystal compound obtained by blending the above compound with the compound of Example 1.

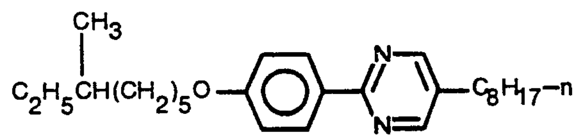

at a proportion of 1:3.

The transition temperature is

$$Cry \underset{-1°C}{\overset{2°C}{\rightleftarrows}} Sm\ C \xrightarrow{*39.6} Sm\ A \xrightarrow{49.7} Iso$$

The response speed is 180 μs (30.0°C).

As is obvious from a comparison with the data of Example 1, it will be appreciated that the liquid crystal compound of this Example has the effect of speeding up the response speed without substantially changing the transition temperature.

Example 42

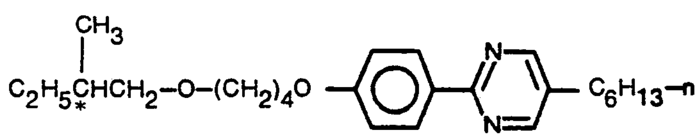

1) Method of synthesizing optically active (s)-alkoxy alcohol (common to all the Examples)

By having active amyl alcohol ($[\alpha]_D^{25} = -4.48°$ (neat)) as starting material and by reacting with appropriate diol in the general method, the following material can be obtained

$$CH_3CH_2\overset{*}{C}H(CH_3)CH_2-O-(CH_2)m-OH-$$

The optically active alcoxy alcohol obtained as above, can easily be transferred into an alphatic or aromatic sulfonic acid ester or into a halogenide compound.

2) Method of synthesizing optically active (s)-5-n-hexyl-2-[4-(2'-methylbutyloxybutyloxy)phenyl]pyrimidine

Pour 16 ml of dry N,N-dimethyl formamide and 0.20 g of sodium hydride (about 50% oil suspension) into a 100 ml four-mouth flask equipped with a cooling tube, dropping funnel, thermometer, and calcium chloride tube. Next, drip 1.61 g of 4-(5-n-hexyl-2-pyrimidinyl)phenol dissolved in 4 ml of dry N,N-dimethyl formamide, Make the mixture react until hydrogen stops generating. Then drip 2.0 g of optically active (s)-p-toluene sulfonic acid 2-methyl butyl oxybutyl ester (this compound is obtained by the active amyl alcohol obtained with the above method 1) into it. Then react for 7 hours at 70 to 80°C. After the reaction, the reaction product is poured into ice water, and on the reacted material, a chloroform extraction is conducted.

The chloroform layer is water-washed and dried, and the chloroform is evaporated and removed. The obtained impure product is repeatedly refined, and 1.2 g of optically active (s)-5-5-n-hexyl-2-[4-(2'-methyl butyloxbutyloxy)phenyl]pyrimidine are obtained having the following characteristics.

IR $\nu_{max}^{film}$ ($cm^{-1}$): 1610, 1585, 1425, 1250, 1170, 1110, 800.

$^1H$-NMR (60 MHz, $CDCl_3$/TMS int) δ (ppm):

8.52 (S, 2H, Pyrimidine H)
8.36 (d, 2H, Aromatic H)
6.94 (d, 2H, Aromatic H)
3.97 (t, 2H, $-CH_2-O-$)
3.39 (t, 2H, $-CH_2-O-$)
3.18 (dd, 2H, $-CH-CH_2-O-$)
2.50 (t, 2H, $-CH_2$-pyr)
0.5~2.2 (m, 24H).

The following result was obtained by measuring the transition temperature (0°C) of the obtained compound

$$Cry \underset{\longleftarrow}{\xrightarrow{-8°C}} Sm\ C^* \xrightarrow{8} Ch \xrightarrow{28} Iso$$

Example 43

Optically active (s)-5-n-octyl-2-[4-(2'-methylbutyloxybutyloxy)phenyl]pyrimidine was synthesized using a similar method to Example 42.

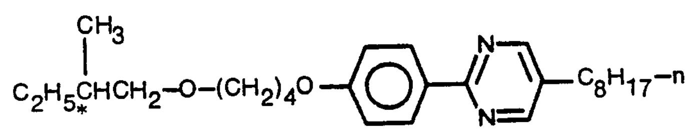

IR $\nu_{max}^{film}$ =: 1610, 1585, 1430, 1250, 1170, 1110, 800.

$^1H$-NMR (60 MHz, $CDCl_3$/TMS int) δ (ppm):

8.50 (S, 2H, Pyrimidine H)
8.40 (d, 2H, Aromatic H)
6.99 (d, 2H, Aromatic H)
4.05 (t, 2H, $-CH_2-O-$)
3.46 (t, 2H, $-CH_2-O-$)
3.24 (dd, 2H, $-CH-CH_2-O-$)
2.58 (t, 2H, $-CH_2$-pyr)
0.5~2.1 (m, 28H).

The following result was obtained by measuring the transition temperature (0°C) of the obtained compound

$$Cry \underset{*-4}{\overset{-1}{\rightleftarrows}} Sm\ C \xrightarrow{*42.0} Iso$$

Example 44

Optically active (s)-5-n-undecyl-2-[4-(2'-methylbutyloxybutyl)phenyl]pyrimidine was synthesized using a similar method to Example 42.

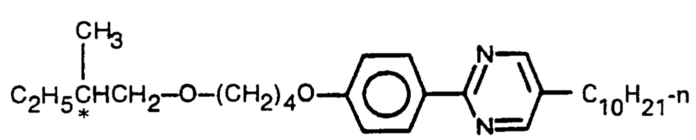

IR $\nu_{max}^{film}$ =: 1610, 1585, 1435, 1260, 1175, 1115, 800.

$^1$H-NMR (60 MHz, $CDCl_3$/TMS int) δ (ppm):

8.57 (S, 2H, Pyrimidine H)
8.36 (d, 2H, Aromatic H)
6.98 (d, 2H, Aromatic H)
4.02 (t, 2H, $—CH_2—O—$)
3.42 (t, 2H, $—CH_2—O—$)
3.20 (dd, 2H, $—CH—CH_2—O—$)
2.55 (t, 2H, $—CH_2$-pyr)
0.5~2.1 (m, 34H).

The following result was obtained by measuring the transition temperature (0°C) of the obtained compound

$$\text{Cry} \underset{*6}{\overset{16}{\rightleftarrows}} \text{Sm C*} \xrightarrow{520} \text{Iso}$$

Example 45

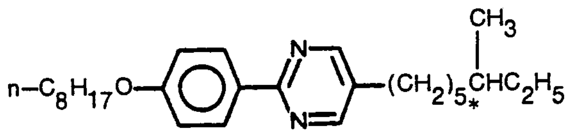

Method of synthesizing active (s)-2-[4-n-octyloxyphenyl]-5-[6-methyloctyl]pyrimidine

Pour 0.33 g of sodium hydride (about 50% oil suspension), and 3 ml of dry N,N-dimethyl formamide into a 30 ml three-mouth flask equipped with a cooling tube, thermometer, dripping funnel, and calcium chloride tube. Next, slowly drip 1.71 g of optically active (s)-4-[5-(6-methyloctyl)-2-pyrimidinyl]phenol which is dissolved in 6 ml of dry N,N-dimethyl formamide at room temperature. After reacting the mixture for 30 minutes, add 1.10 g of 1-bromoctane, and react for 7 hours at 90°C. After the reaction, pour the reaction product into ice water, then extract the product with ethyl acetate. The organic layer is thoroughly washed with water and dried. The organic solvent is evaporated and removed, and then an impure product is obtained. This impure product is refined with silica gel chromatography and re-crystallized crystallization to obtain 1.3 g of optically active (s)-2-[4-n-octyloxyphenyl]-5-[6-methyloctyl]pyrimidine having the following characteristics.

IR ν max ($cm^{-1}$): 1610, 1585, 1430, 1250, 1170, 800.

$^1$H-NMR (60 MHz, $CDCl_3$) δ (ppm):

8.38 (S, 2H)
8.28 (d, 2H)
6.97 (d, 2H)
3.27~4.20 (m, 4H)
0.6~2.1 (m, 32H).

$$\text{Cry} \underset{6°C}{\overset{14°C}{\rightleftarrows}} \text{Sm C} \underset{29.4°C}{\overset{*28°C}{\rightleftarrows}} \text{Ch} \underset{43.5°C}{\overset{44°C}{\rightleftarrows}} \text{Iso}$$

Example 46

Method of synthesizing (s)-2-(4-n-octylphenyl)-5-(6-methyloctyloxy)pyrimidine.

Pour 2.64 g of 4-n-octyl benzamidine hydrochloric acid salt, 2.38 g (s)-α-(6-methyloctyloxy)-β-(dimethylamino) acrolein, and 20 ml ethanol anhydride into a three-mouth flask equipped with a cooling tube, calcium chloride tube, dripping funnel, and thermometer. Next, slowly drip in 4.18 g of 28% sodium methylate methanol solution at room temperature. After the dripping, conduct a reflux action and leave to react for 11 hours. When the reaction finishes, pour the reaction product into ice water. Then extract the reaction product with ethyl acetate.

The ethyl acetate layer is water washed and dried. The solvent is evaporated and removed at low pressure. The obtained impure product is refined with silica gel carame chromatography, and recrystallized crystal. Then 2.41 g of (s)-2-[4-n-octylphenyl]-5-(6-methyloctyloxy)pyrimidine are obtained having the following characteristics.

IR ν max ($cm^{-1}$): 1610, 1578, 1440, 1280, 785.

$^1$H-NMR (60 MHz, $CDCl_3$) δ (ppm):

0.65~2.2 (m, 32H)
2.66 (t, 2H)
4.04 (t, 2H)
7.30 (d, 2H)
8.30 (d, 2H)
8.45 (s, 2H)

$$\text{Cry} \underset{-22°C}{\overset{-13°C}{\rightleftarrows}} \text{Sm X} \underset{5°C}{\overset{10°C}{\rightleftarrows}} \text{Sm Y} \underset{9.5°C}{\overset{18°C}{\rightleftarrows}} {}^{*}\text{Sm C} \underset{50°C}{\overset{51°C}{\rightleftarrows}} \text{Sm A} \underset{51.4°C}{\overset{51.4°C}{\rightleftarrows}} \text{Iso}$$

(here, Sm X, Sm Y are smectic phases of which phase is unconfirmed).

Example 47

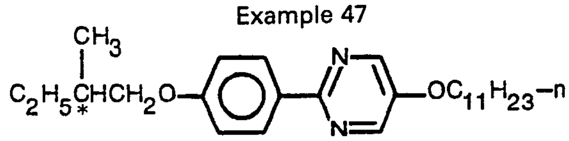

Method of synthesizing (s)-5-(n-undecyloxy)-2-[4-(2-methylbutyloxy)phenyl]pyrimidine.

Suspend 0.185 g of 50% sodium hydride in 2 ml dry DMF. Slowly drip in 1.1 g of 2-(4-hydroxyphenyl)-5-(n-undecyl)pyrimidine which is resolved in 5 ml dry DMF at a temperature below freezing point. Agitate the mixture for 30 minutes at room temperature, then agitate for another 30 minutes at room temperature. Then drip 0.785 g of 2-methyl-1-(p-tolylsulfonyloxy)butane (this is regulated by (s)-amyl alcohol ($[\alpha]_D^{23}$ −5.8° (neat)). Agitate the reaction compound solution for 5 hours at 100°C and then pour it into ice water. Extract the reaction product with ethyl ester acetate.

The ethyl ester acetate layer is washed with 5% of NaOH, dried with magnesium sulfate. The remaining sediment is obtained by evaporating and removing the solvent. The sediment is then refined with silica gel carame chromatography and re-crystallized crystal. There is then obtained (s)-5-n-(n-undecyloxy)-2-[4-(2-methylbutyloxy)phenyl]pyrimidine, at a yield of 76%.

Examples 48—50

With the compound shown in the following general formula, a compound was synthesized wherein m and n were changed in a similar method as in Example 47.

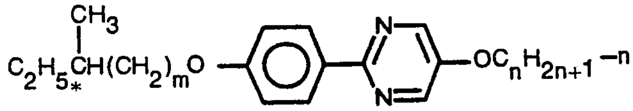

The characteristics are all shown in the following table, taken in conjunction with Example 47.

TABLE 4
Characteristic Measure Value of the Compounds

| object compound | IR τ max $cm^{-1}$ | $^1$H—NMR | |
|---|---|---|---|
| Example 47<br>m = 1<br>n = 11 | 1615, 1590, 1550,<br>1525, 1445, 1250 | 0.7 ~ 2.2<br>3.85<br>4.04<br>7.00<br>8.33<br>8.42 | (m, 30H)<br>(d, 2H)<br>(t, 2H)<br>(d, 2H)<br>(d, 2H)<br>(s, 2H) |
| Example 48<br>m = 2<br>n = 8 | 1612, 1585, 1555,<br>1512, 1440 | 0.7 ~ 2.1<br>4.05<br>6.95<br>8.28<br>8.36 | (m, 27H)<br>(t, 4H)<br>(d, 2H)<br>(d, 2H)<br>(s, 2H) |
| Example 49<br>m = 5<br>n = 8 | 1610, 1584, 1525,<br>1515, 1435 | 0.6 ~ 2.1<br>2.60<br>4.00<br>6.97<br>8.36<br>8.57 | (m, 32H)<br>(t, 2H)<br>(t, 2H)<br>(d, 2H)<br>(d, 2H)<br>(s, 2H) |
| Example 50<br>m = 2<br>n = 11 | 1610, 1510, 1550,<br>1512, 1440 | 0.7 ~ 2.27<br>4.13<br>7.02<br>8.35<br>8.45 | (m, 32H)<br>(t, 4H)<br>(d, 2H)<br>(d, 2H)<br>(s, 2H) |

TABLE 5

The Phase Transition Temperature and the Response Speed of the Compound

| object compound | transition temperature | response speed |
|---|---|---|
| Example 47 m = 1 n = 11 | $Cry \underset{*44}{\overset{58}{\rightleftarrows}} Sm\ C \xrightarrow{*72.7} Sm\ A \xrightarrow{76.7} Iso$ | τ = 290 μs (66°C) |
| Example 48 m = 2 n = 8 | $Cry \underset{*}{\overset{52.4}{\rightleftarrows}} Sm\ C \xrightarrow{*73.1} Sm\ A \xrightarrow{76.5} Iso$ | τ = 900 μs (65°C) |
| Example 49 m = 5 n = 11 | $Cry \underset{*}{\overset{40.7}{\rightleftarrows}} Sm\ C \xrightarrow{*82.8} Sm\ A \xrightarrow{89.1} Iso$ | τ = 1.5 ms (43°C) |
| Example 50 m = 2 n = 11 | $Cry \underset{*32.3}{\overset{48.5}{\rightleftarrows}} Sm\ C \xrightarrow{*78.0} Iso$ | τ = 310 μs (70°C) |

(here, * stands for supercooling).

Example 51

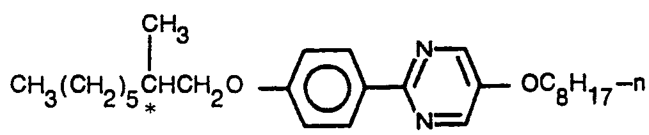

(s)-5-(n-octyloxy)-2-[4-(2-methyloctyl)phenyl]pyrimidine was synthesized using a similar method to Example 47.

The response speed was measured using a similar method to the transition temperature, and is shown as follows.

Transition temperature

$$Cry \underset{*26}{\overset{36.2}{\rightleftarrows}} Sm\ C^* \xrightarrow{40.8} Sm\ A \xrightarrow{62.4} Iso$$

Response speed: 300 μs (30°C)

Example 52

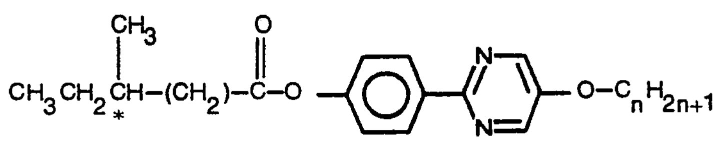

(m = 0, n = 11, * asymmetric carbon)

Method of synthesizing (s)-5-n-undecyloxy-2-[4-(2-methylbutanoyloxyphenyl]pyrimidine

Pour 0.328 g of 2-methyl butyric acid synthesized from (s)-amylalcohol ($[\alpha]_D^{23}$ −5.8° (neat)), 1.1 g of 4-(5-n-undecyloxy-2-pyrimidinyl)phenol, 8 ml of anhydride chloroform and 0.662 g of 4-dimethyl amino pyridine, N—N dicyclohexyl carbodiimido, into 25 ml flask. The mixture is caused to react for one whole day at room temperature. After the reaction, filter and separate the insoluble matter, and conduct a chloroform extraction on the reaction product. The organic layer is washed with water, 2N hydrochloric acid and water, and dried. Then evaporate and remove the organic solvent. Refine the obtained reaction product with silica gel chromatography and re-crystallized crystal. Then optically active (s)-5-n-undecyloxy-2-4-(2-methylbutanoyloxy)phenyl]pyrimidine is obtained at the yield of 70% and with the following characteristics.

IR $\nu_{max}^{nujol}$ ($cm^{-1}$): 1762, 1602, 1550, 1440, 1278.

$^1$H-NMR (60 MHz, $CDCl_3$) δ (ppm):

8.42 (S, 2H)
8.40 (d, 2H)
7.17 (d, 2H)
4.40 (t, 2H)
2.3~2.8 (m, 1H)
0.6~2.2 (m, 29H)

Examples 53—57

An object compound was obtained using a similar method to Example 1, utilizing the (s) form alkyl acid synthesized from (s) amyl alcohol $[\alpha]_D^{-23}$ −5.8° (neat), and alkyloxypyrimidinylphenol. Refer to the following table.

TABLE 6—1

| Example | starting material | | | | | object compound | | |
|---|---|---|---|---|---|---|---|---|
| | m | 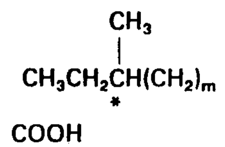 | n | 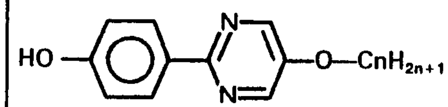 | yield (%) | 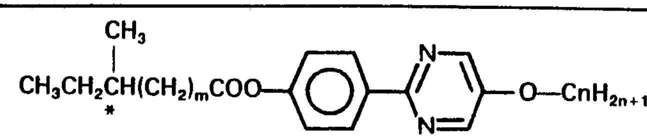 | | |
| | | | | | | m<br>n | I.R.<br>ν nujol<br>max ($cm^{-1}$) | H—N.M.R. (60 MHZ, $CDCl_3$)<br>δ (ppm) |
| 53 | 0 | 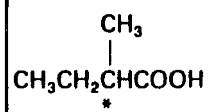 | 8 | 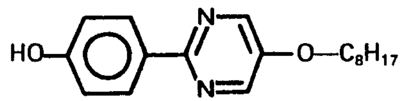 | 60 | m = 0<br>n = 8 | 1760<br>1555<br>1440<br>1200<br>1205<br>1160 | 8.47 (S, 2H)<br>8.37 (d, 2H)<br>7.17 (d, 2H)<br>4.17 (t, 2H)<br>2.35 ~ 3.04 (m, 1H)<br>0.77 ~ 2.35 (m, 23H) |
| 54 | 1 | 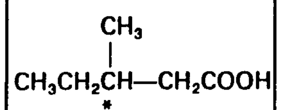 | 8 | 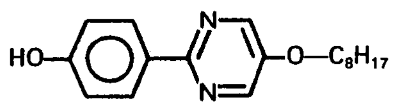 | 77 | m = 1<br>n = 8 | 1762<br>1598<br>1560<br>1450<br>1290 | 8.41 (S, 2H)<br>8.36 (d, 2H)<br>7.16 (d, 2H)<br>4.08 (t, 2H)<br>2.31 ~ 2.85 (m, 2H)<br>0.7 ~ 2.25 (m, 24H) |
| 55 | 2 | 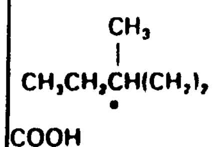 | 8 | 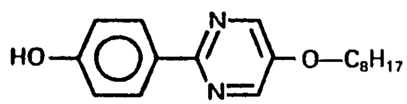 | 66 | m = 2<br>n = 8 | 1762<br>1598<br>1560<br>1450<br>1290 | 8.43 (S, 2H)<br>8.42 (d, 2H)<br>7.21 (d, 2H)<br>4.05 (t, 2H)<br>2.58 (t, 2H)<br>0.6 ~ 2.1 (m, 30H) |

TABLE 6 continued

| | | starting material | | | | object compound | | |
|---|---|---|---|---|---|---|---|---|
| 56 | 4 | $CH_3$<br>\|<br>$CH_3CH_2CH(CH_2)_4$<br>*<br>COOH | 8 | 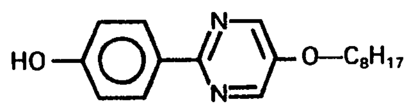 | 75 | m = 4<br>n = 8 | 1762<br>1598<br>1560 | 8.44 (S, 2H)<br>8.40 (d, 2H)<br>7.19 (d, 2H)<br>4.06 (t, 2H)<br>2.58 (t, 2H)<br>0.65 ~ 2.15 (m, 30H) |
| 57 | 1 | $CH_3$<br>\|<br>$CH_3CH_2CH—CH_2$<br>*<br>COOH | 11 | 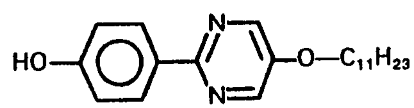 | 66 | m = 1<br>n = 11 | 1760<br>1598<br>1560<br>1450<br>1290 | 8.42 (S, 2H)<br>8.35 (d, 2H)<br>7.27 (d, 2H)<br>4.08 (t, 2H)<br>2.32 ~ 2.82 (m, 2H)<br>0.68 ~ 2.27 (m, 30H) |

TABLE 7

The Transition Temperature and the Response Speed of the Compounds

| object compound | phase transition temperature | response speed |
|---|---|---|
| Example 52<br>m = 0<br>n = 11 | Cry ⇄ Sm C (43 →, ← *42)<br>Sm C —*64.1→ Sm A —67.2→ Iso | τ = 230 μs<br>(60°C) |
| Example 53<br>m = 0<br>n = 8 | Cry —66.0→ Iso<br>Iso —62.2→ Sm C*<br>Sm C* —*56.2→ Cry | τ = 150 μs<br>(62°C) |
| Example 54<br>m = 1<br>n = 8 | Cry ⇄ Sm C (70 →, ← *59.8)<br>Sm C —*72.2→ Sm A —72.9→ Iso | τ = 250 μs<br>(66°C) |
| Example 55<br>m = 2<br>n = 8 | Cry —78.0→ Iso<br>Iso —75.5→ Sm C*<br>Sm C* —70.2→ Cry | — |
| Example 56<br>m = 4<br>n = 8 | Cry ⇄ Sm C (76.0 →, ← *64.7)<br>Sm C —*79.5→ Iso | τ = 1.3 ms<br>(78°C) |
| Example 57<br>m = 1<br>n = 11 | Cry —54.8→ Sm C —*72.3→ Sm A —77.8→ Iso<br>Sm C → $S_1$ —33.7→ $S_2$ —*→ Cry | τ = 240 μs<br>(60°C) |

* shows supercooling.

Examples 58—61

The data of the structural formula and the transition temperature of synthesized specific compounds is shown in the following Table 8.

TABLE 8

| Example No. | structural formula | phase transition temperature (°C) |
|---|---|---|
| 58 | 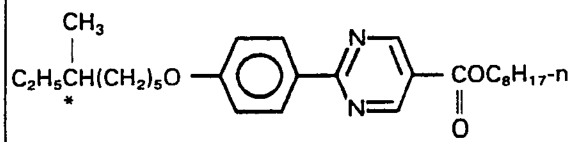 | $Cry \underset{*}{\overset{78.5}{\rightleftarrows}} Sm\ C \xrightarrow{*79.7} Iso$ |
| 59 | 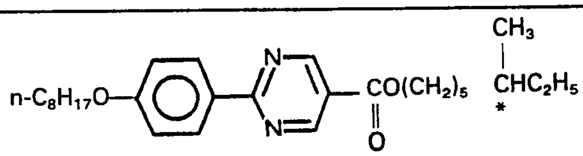 | $Cry \underset{*}{\overset{79.9}{\rightleftarrows}} Sm\ C \xrightarrow{*80.7} Iso$ |
| 60 | 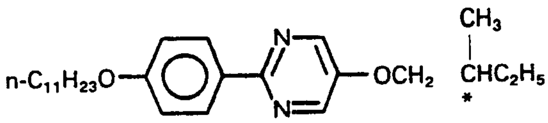 | $Cry \underset{*41.0}{\overset{51.0}{\rightleftarrows}} Iso$ |
| 61 | 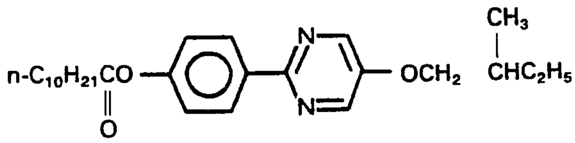 | $Cry \underset{*51.0}{\overset{58.2}{\rightleftarrows}} Iso$ |

Example 62

Method of synthesizing (R)-5-n-undecyl-2-[4-(2'-methylbutyloxy)phenyl]pyrimidine

Suspend 0.57 g of 50% of sodium hydride in 6 ml of dried dimethyl formamide. Dissolve and add 3.2 g of 2-(4-hydroxyphenyl)-5-n-undecyl pyrimidine in 6 ml dry dimethyl formamide. Agitate the mixture for 60 minutes at room temperature. Dissolve and add 2.9 g (R)-2-methyl-1-(p-toluenesulfonyl)oxbuthane in 5 ml dry dimethyl formamide. Heat the mixture to 75 to 80°C and agitate for 8 hours. Then pour the reacted solution into ice water, extract it with ethyl acetate, wash it with salt water, and dry it with magnesium sulfate. Evaporate and remove the solvent in order to obtain 4.27 g of remained sediment. Refine this with silica gel carame chromatography and re-crystallization. There is then obtained 2.0 g of (R)-5-n-undecyl-2-[4-(2'-methylbutyloxy)phenyl]pyrimidine having the following characteristics.

$[\alpha]_D^{25}$ −7.05 (C = 2.0, $CHCl_3$)

I.R. ν max ($cm^{-1}$): 1610, 1590, 1435, 1255, 1170, 850, 800.

$^1$H-NMR (60 MHz, $CDCl_3$) δ (ppm):

0.6~2.23 (m, 30H)
2.53 (t, 2H)
3.72 (d, 2H)
6.88 (d, 2H)
8.24 (d, 2H)
8.43 (s, 2H).

The transition temperature of this liquid crystal compound is as follows.

$$Cry \underset{*}{\overset{35.0}{\rightleftarrows}} Sm\ C \xrightarrow{*38.2} Sm\ A \xrightarrow{49.0} Iso$$

This liquid crystal compound is sandwiched between PVA rubbing one-axis orientation processed plates. The liquid crystal layer thickness is fixed at 2.5 um, and ±20V voltage is applied. The characteristic at orthogonal nicol is measured. The measuring temperature is 35°C.

The response speed is 210 μs.

Example 63

Method of synthesizing (R)-5-n-octyl-2-[4-(4'-methylhexyloxy)phenyl]pyrimidine

Suspend 0.605 g of 59% sodium hydride in 5 ml dry dimethyl formamide. Dissolve and add 3.0 g 2-(4-hydroxyphenyl)-5-n-octyl pyrimidine in 7 ml dry dimethyl formamide. Agitate for 60 minutes at room temperature. Dissolve and add 2.84 g of (R)-4-methyl-1-(p-toluensulfonyl)oxy hexane in 5 ml dry dimethyl formamide. Heat the compound to 75 to 80°C, and agitate for 8 hours. Pour the reaction product in ice water, extract it with ethyl acetate, wash with salt water, dry it with magnesium sulfate, evaporate and remove the solvent. There is then obtained 4.15 g of remaining sediment. Refine this sediment with silica gel carame chromatography and re-crystallization. Thee are then obtained 2.5 g of (R)-5-n-octyl-2-[4-(4'-methylhexyloxy)phenyl]pyrimidine having the following characteristics.

$[\alpha]_D^{25}$ −5.25° (C = 2, $CHCl_3$).

I.R. ν max ($cm^{-1}$): 1610, 1590, 1435, 1255, 1170, 850, 802.

$^1$H-NMR (60 MHz, $CDCl_3$) δ (ppm):

0.6~2.20 (m, 28H)
2.55 (t, 2H)
3.97 (t, 2H)
6.91 (d, 2H)
8.25 (d, 2H)
8.48 (s, 2H).

The transition temperature of this liquid crystal compound is as follows:

$$Cry \underset{*}{\overset{34.0}{\rightleftarrows}} Sm\ C^* \xrightarrow{44.2} Sm\ A \xrightarrow{51.5} Iso$$

* shows supercooling.

This liquid crystal compound is sandwiched between PVA rubbing one-axis orientation processed plates. The liquid crystal layer thickness is fixed at 2.5 μm, and ±20V of voltage is applied. Its characteristics under orthogonal nicol is measured. The measured temperature is 27°C.

The response speed is 245 μs.

It will be appreciated from the above description referring to the drawings and the Examples, that the new liquid crystal compounds of the present invention have a very good response ability. They are able to have a Sm C* phase over a wide temperature range close to room temperature. Thus the liquid crystal compounds have a practical Sm C* phase temperature range which makes them first-rate industrial materials for example for use in ferroelectric liquid crystal displays.

## Claims

1. A ferroelectric smectic liquid crystal compound having the general formula

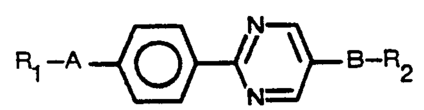

wherein:

A and B respectively are

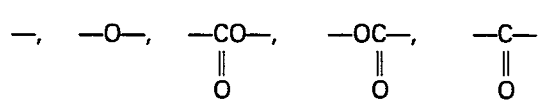

(— is a direct bond);

one of $R_1$ and $R_2$ is a straight chain alkyl group, and the other is either an alkyl group having an asymmetric carbon atom or an alkoxy alkyl group having an asymmetric carbon atom and ether bonding; and

onto the asymmetric carbon atom there is added one of the groups $—CH_3$, —CN, —Cl.

2. A ferroelectric smectic liquid crystal compound as claimed in claim 1 having the formula

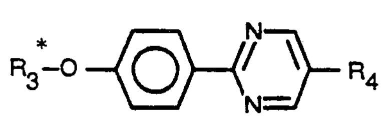

wherein $R_3$* is an alkyl group that has an asymmetric carbon atom and $R_4$ is a straight chain alkyl group.

3. A ferroelectric smectic liquid crystal compound as claimed in claim 1 having the formula

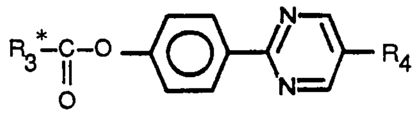

wherein $R_3$* is an alkyl group having an asymmetric carbon atom, and $R_4$ is a straight chain alkyl group.

4. A ferroelectric smectic liquid crystal compound as claimed in claim 1 having the formula

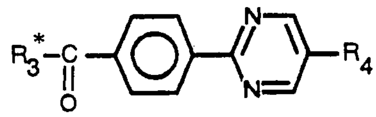

wherein $R_3$* is an alkyl group having an asymmetric carbon atom, and $R_4$ is a straight chain alkyl group.

5. A ferroelectric smectic liquid crystal compound as claimed in claim 1 having the formula

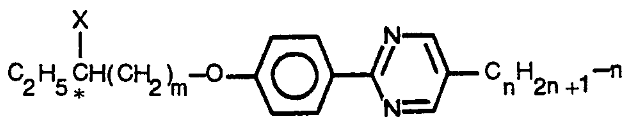

wherein m is an integral number from 0 to 8, n is an integral number from 5 to 14, X is either —Cl or —CN, * is an asymmetric carbon atom, and the alkyl group containing —n being a straight chain.

6. A ferroelectric smectic liquid crystal compound as claimed in claim 1 having the formula

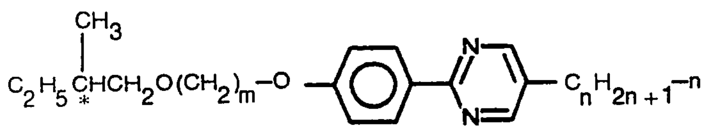

wherein m is an integral number from 1 to 8, n is an integral number from 5 to 14, * is an asymmetric carbon atom, and the alkyl group containing —n being a straight chain.

7. A ferroelectric smectic liquid crystal compound as claimed in claim 1 having the formula

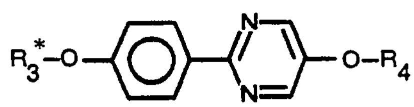

wherein $R_3$* is an alkyl group having an asymmetric carbon atom, and $R_4$ is a straight chain alkyl group.

8. A ferroelectric smectic liquid crystal compound as claimed in claim 1 having the formula

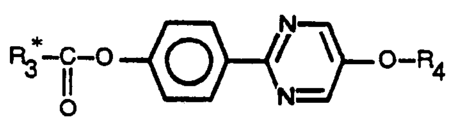

wherein $R_3$* is an alkyl group having an asymmetric carbon atom, and $R_4$ is a straight chain alkyl group.

9. A ferroelectric smectic liquid crystal compound as claimed in claim 1 having the formula

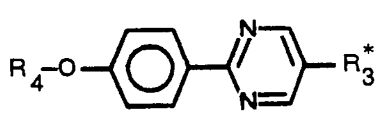

wherein $R_3$* is an alkyl group having an asymmetric carbon atom, and $R_4$ is a straight chain alkyl group.

10. A ferroelectric smectic liquid crystal compound as claimed in claim 1 having the formula

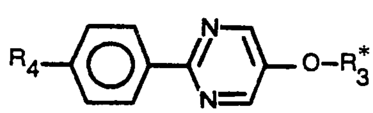

wherein $R_3$* is an alkyl group having an asymmetric carbon atom, and $R_4$ is a straight chain alkyl group.

11. A ferroelectric smectic liquid crystal compound as claimed in claim 1 having the formula

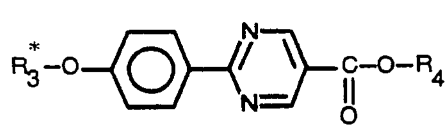

wherein $R_3$* is an alkyl group having an asymmetric carbon atom, and $R_4$ is a straight chain alkyl group.

12. A ferroelectric smectic liquid crystal compound as claimed in claim 1 having the formula

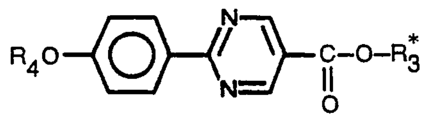

wherein $R_3$* is an alkyl group having an asymmetric carbon atom, and $R_4$ is a straight chain alkyl group.

13. A ferroelectric smectic liquid crystal compound as claimed in claim 1 having the formula

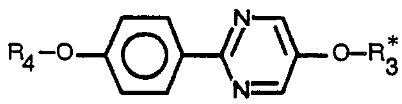

wherein $R_3$* is an alkyl group having an asymmetric carbon atom, and $R_4$ is a straight chain alkyl group.

14. A ferroelectric smectic liquid crystal compound as claimed in claim 1 having the formula

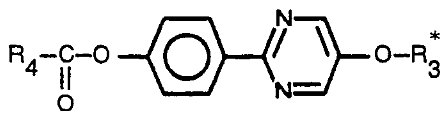

wherein $R_3$* is an alkyl group having an asymmetric carbon atom, and $R_4$ is a straight chain alkyl group.

15. An additive compound for a ferroelectric smectic liquid crystal mixture, having the general formula

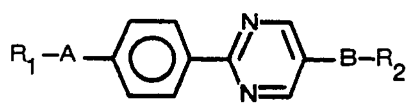

wherein:

A and B respectively are

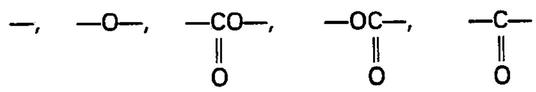

(— is a direct bond);

one of $R_1$ and $R_2$ is a straight chain alkyl group, and the other is either an alkyl group having an asymmetric carbon atom or an alkoxy alkyl group having an asymmetric carbon atom and ether bonding; and

onto the asymmetric carbon atom there is added one of the groups $—CH_3$, —CN, —Cl.

16. An additive compound for a ferroelectric smectic liquid crystal mixture as claimed in claim 15, having the formula:

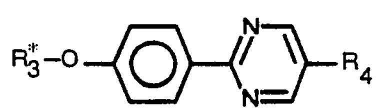

wherein $R_3$* is an alkyl group that has an asymmetric carbon atom, and $R_4$ is a straight chain alkyl group.

17. An additive compound for a ferroelectric smectic liquid crystal mixture as claimed in claim 15, having the formula:

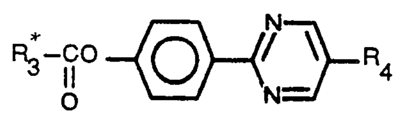

wherein $R_3$* is an alkyl group having an asymmetric carbon atom, and $R_4$ is a straight chain alkyl group.

18. An additive compound for a ferroelectric smectic liquid crystal mixture as claimed in claim 15, having the formula:

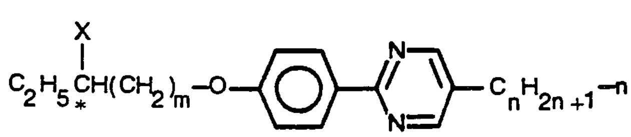

wherein m is an integral number from 0 to 8, n is an integral number from 5 to 14, X is either —Cl or —CN, * is an asymmetric carbon atom, and the alkyl group containing —n being a straight chain.

19. An additive compound for a ferroelectric smectic liquid crystal mixture as claimed in claim 15, having the formula:

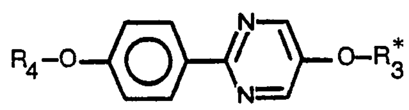

wherein $R_3$* is an alkyl group having an asymmetric carbon atom, and $R_4$ is a straight chain alkyl group.

20. An additive compound for a ferroelectric smectic liquid crystal mixture as claimed in claim 15, having the formula:

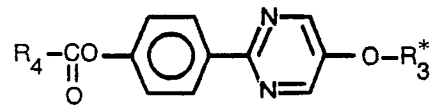

wherein $R_3$* is an alkyl group having an asymmetric carbon atom, and $R_4$ is a straight chain alkyl group.

**Patentansprüche**

1. Ferroelektrische smektische Flüssigkristallverbindung mit der allgemeinen Formel

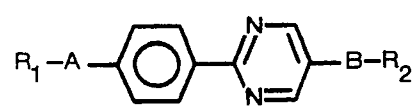

worin:

A bzw. B 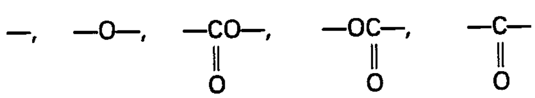 sind

(und — eine direkte Bindung bedeutet),

$R_1$ oder $R_2$ eine geradkettige Alkylgruppe ist, und das jeweils andere Symbol entweder eine Alkylgruppe mit einem asymmetrischen Kohlenstoffatom und Etherbindung bedeutet, und

sich eine der Gruppen $—CH_3$, —CN, —Cl am asymmetrischen Kohlenstoffatom befindet.

2. Ferroelektrische smektische Flüssigkristallverbindung nach Anspruch 1 mit der Formel

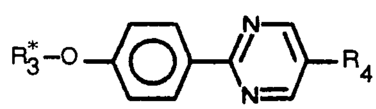

worin $R_3$* eine Alkylgruppe mit einem asymmetrischen Kohlenstoffatom und $R_4$ eine geradkettige Alkylgruppe ist.

3. Ferroelektrische smektische Flüssigkristallverbindung nach Anspruch 1 mit der Formel

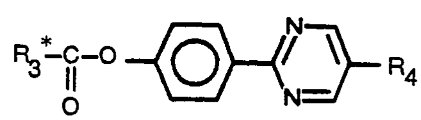

worin $R_3$* eine Alkylgruppe mit einem asymmetrischen Kohlenstoffatom und $R_4$ eine geradkettige Alkylgruppe ist.

4. Ferroelektrische smektische Flüssigkristallverbindung nach Anspruch 1 mit der Formel

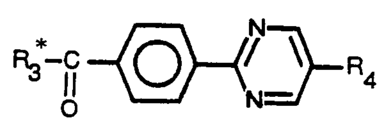

worin $R_3$* eine Alkylgruppe mit einem asymmetrischen Kohlenstoffatom und $R_4$ eine geradkettige Alkylgruppe ist.

5. Ferroelektrische smektische Flüssigkristallverbindung nach Anspruch 1 mit der Formel

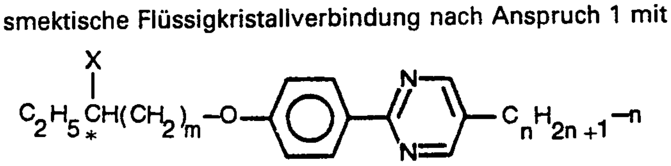

worin m eine ganze Zahl von 0 bis 8 ist, n eine ganze Zahl von von 5 bis 14 ist, X entweder —Cl oder —CN ist, * ein asymmetrisches Kohlenstoffatom ist und die —n enthaltende Alkylgruppe eine gerade Kette ist.

6. Ferroelektrische smektische Flüssigkristallverbindung nach Anspruch 1

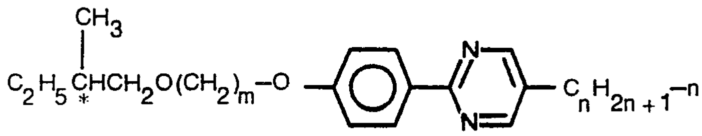

worin m eine ganze Zahl von 1 bis 8 ist, n eine ganze Zahl von von 5 bis 14 ist, * ein asymmetrisches Kohlenstoffatom ist und die —n enthaltende Alkylgruppe eine gerade Kette ist.

7. Ferroelektrische smektische Flüssigkristallverbindung nach Anspruch 1 mit der Formel

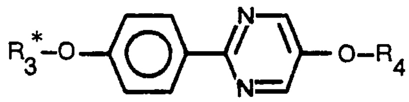

worin $R_3$* eine Alkylgruppe mit einem asymmetrischen Kohlenstoffatom und $R_4$ eine geradkettige Alkylgruppe ist.

8. Ferroelektrische smektische Flüssigkristallverbindung nach Anspruch 1

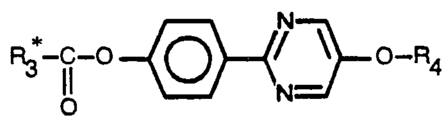

worin $R_3$* eine Alkylgruppe mit einem asymmetrischen Kohlenstoffatom und $R_4$ eine geradkettige Alkylgruppe ist.

9. Ferroelektrische smektische Flüssigkristallverbindung nach Anspruch 1

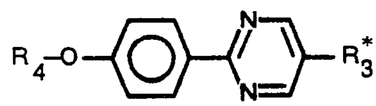

worin $R_3$* eine Alkylgruppe mit einem asymmetrischen Kohlenstoffatom und $R_4$ eine geradkettige Alkylgruppe ist.

10. Ferroelektrische smektische Flüssigkristallverbindung nach Anspruch 1

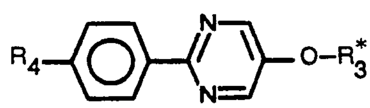

worin $R_3$* eine Alkylgruppe mit einem asymmetrischen Kohlenstoffatom und $R_4$ eine geradkettige Alkylgruppe ist.

11. Ferroelektrische smektische Flüssigkristallverbindung nach Anspruch 1

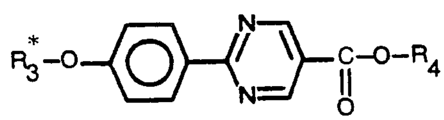

worin $R_3$* eine Alkylgruppe mit einem asymmetrischen Kohlenstoffatom und $R_4$ eine geradkettige Alkylgruppe ist.

12. Ferroelektrische smektische Flüssigkristallverbindung nach Anspruch 1

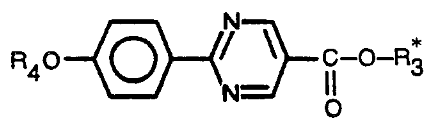

worin $R_3$* eine Alkylgruppe mit einem asymmetrischen Kohlenstoffatom und $R_4$ eine geradkettige Alkylgruppe ist.

13. Ferroelektrische smektische Flüssigkristallverbindung nach Anspruch 1 mit der formel

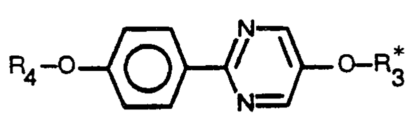

worin $R_3$* eine Alkylgruppe mit einem asymmetrischen Kohlenstoffatom und $R_4$ eine geradkettige Alkylgruppe ist.

14. Ferroelektrische smektische Flüssigkristallverbindung nach Anspruch 1 mit der formel

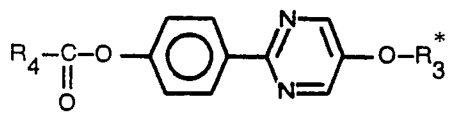

worin $R_3$* eine Alkylgruppe mit einem asymmetrischen Kohlenstoffatom und $R_4$ eine geradkettige Alkylgruppe ist.

15. Zusatzverbindung für ein ferroelektrisches smektisches Flüssigkristallgemisch mit der allgemeinen Formel

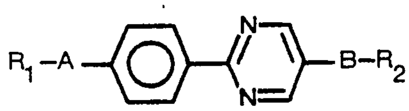

worin:

A bzw. B

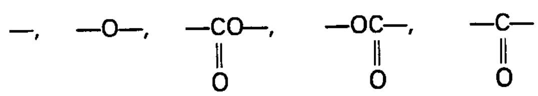 sind

(und — eine direkte Bindung bedeutet),

$R_1$ oder $R_2$ eine geradkettige Alkylgruppe ist, und das jeweils andere Symbol entweder eine Alkylgruppe mit einem asymmetrischen Kohlenstoffatom oder eine Alkoxy-Alkylgruppe mit einem asymmetrischen Kohlenstoffatom und Etherbindung bedeutet, und

sich eine der Gruppen $—CH_3$, —CN, —Cl am asymmetrischen Kohlenstoffatom befindet.

16. Zusatzverbindung für ein ferroelektrisches smektisches Flüssigkristallgemisch nach Anspruch 15 mit der allgemeinen Formel

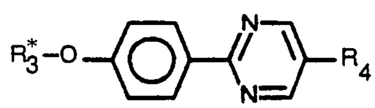

worin $R_3$* eine Alkylgruppe mit einem asymmetrischen Kohlenstoffatom und $R_4$ eine geradkettige Alkylgruppe ist.

17. Zusatzverbindung für ein ferroelektrisches smektisches Flüssigkristallgemisch nach Anspruch 15 mit der allgemeinen Formel

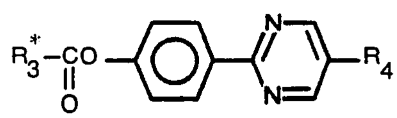

worin $R_3$* eine Alkylgruppe mit einem asymmetrischen Kohlenstoffatom und $R_4$ eine geradkettige Alkylgruppe ist.

18. Zusatzverbindung für ein ferroelektrisches smektisches Flüssigkristallgemisch nach Anspruch 15 mit der allgemeinen Formel

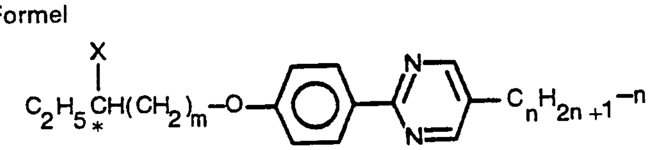

worin m eine ganze Zahl von 0 bis 8 ist, n eine ganze Zahl von von 5 bis 14 ist, X entweder —Cl oder —CN ist, * ein asymmetrisches Kohlenstoffatom ist und die —n enthaltende Alkylgruppe eine gerade Kette ist.

19. Zusatzverbindung für ein ferroelektrisches smektisches Flüssigkristallgemisch nach Anspruch 15 mit der allgemeinen Formel

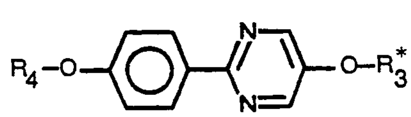

worin $R_3$* eine Alkylgruppe mit einem asymmetrischen Kohlenstoffatom und $R_4$ eine geradkettige Alkylgruppe ist.

20. Zusatzverbindung für ein ferroelektrisches smektisches Flüssigkristallgemisch nach Anspruch 15 mit der allgemeinen Formel

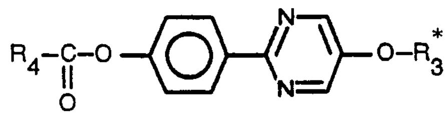

worin $R_3$* eine Alkylgruppe mit einem asymmetrischen Kohlenstoffatom und $R_4$ eine geradkettige Alkylgruppe ist.

**Revendications**

1. Cristal liquide ferroélectrique smectique de formule développée

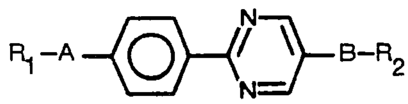

dans laquelle A et B sont respectivement,

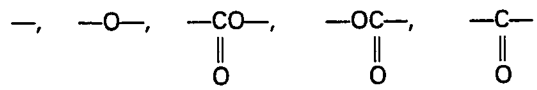

(— est une liaison directe);

l'un de $R_1$ et de $R_2$ est un groupe alcoyle à chaîne linéaire, et l'autre est soit un groupe alcoyle ayant un atome de carbone asymétrique, soit un group alcoxyalcoyle ayant un atome de carbone asymètrique et une liaison éther; et

sur l'atome de carbone asymétrique est ajouté l'un des groupes $—CH_3$, —CN, —Cl.

2. Cristal liquide ferroélectrique smectique suivant la revendication 1 de formule

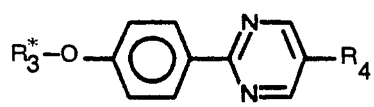

dans laquelle $R_3$* est un groupe alcoyle qui a un atome de carbone asymétrique et $R_4$ est un groupe alcoyle à chaîne linéaire.

3. Cristal liquide ferroélectrique smectique suivant la revendication 1 de formule

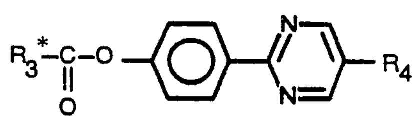

dans laquelle $R_3$* est un groupe alcoyle ayant un atome de carbone asymétrique et $R_4$ est un groupe alcoyle à chaîne linéaire.

4. Cristal liquide ferroélectrique smectique suivant la revendication 1 de formule

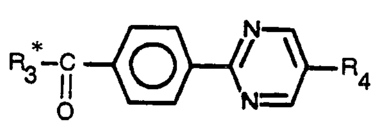

dans laquelle $R_3$* est un groupe alcoyle ayant un atome de carbone asymétrique et $R_4$ est un groupe alcoyle à chaîne linéaire.

5. Cristal liquide ferroélectrique smectique suivant la revendication 1 de formule

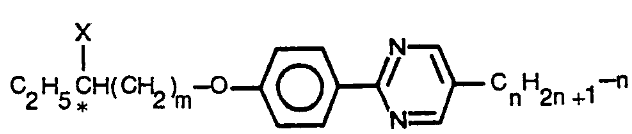

dans laquelle m est un nombre entier allant de 0 à 8, n est un nombre entier allant de 5 à 14, X est —Cl ou —CN, C* un atome de carbone asymétrique et le groupe alcoyle contenant —n est une chaîne linéaire.

6. Cristal liquide ferroélectrique smectique suivant la revendication 1 de formule

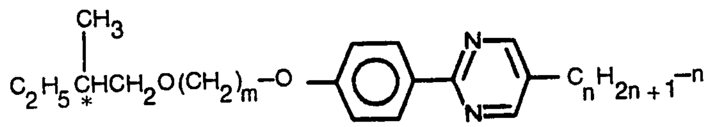

dans laquelle m est un nombre entier allant de 1 à 8, n est un nombre entier allant de 7 à 14, C* est un atome de carbone asymétriaue et le groupe alcoyle contenant —n est une chaîne linéaire.

7. Cristal liquide ferroélectrique smectique suivant la revendication 1 de formule

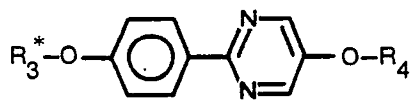

dans laquelle $R_3$* est un groupe alcoyle ayant un atome de carbone asymétrique et $R_4$ est un groupe alcoyle à chaîne linéaire.

8. Cristal liquide ferroélectrique smectique suivant la revendication 1 de formule

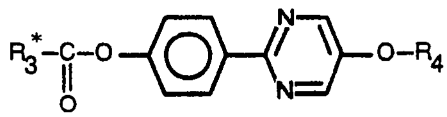

dans laquelle $R_3$* est un groupe alcoyle ayant un atome de carbone asymétrique et $R_4$ est un groupe alcoyle à chaîne linéaire.

9. Cristal liquide ferroélectrique smectique suivant la revendication 1 de formule

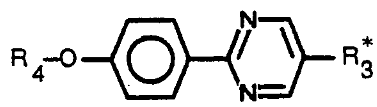

dans laquelle $R_3$* est un groupe alcoyle ayant un atome de carbone asymétrique et $R_4$ est un groupe alcoyle à chaîne linéaire.

10. Cristal liquide ferroélectrique smectique suivant la revendication 1 de formule

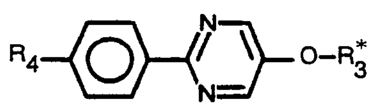

dans laquelle $R_3$* est un groupe alcoyle ayant un atome de carbone asymétrique et $R_4$ est un groupe alcoyle à chaîne linéaire.

11. Cristal liquide ferroélectrique smectique suivant la revendication 1 de formule

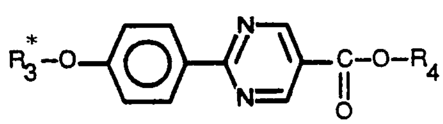

dans laquelle $R_3$* est un groupe alcoyle ayant un atome de carbone asymétrique et $R_4$ est un groupe alcoyle à chaîne linéaire.

12. Cristal liquide ferroélectrique smectique suivant la revendication 1 de formule

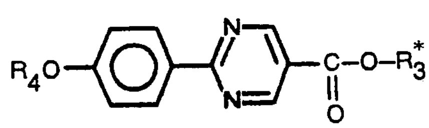

dans laquelle $R_3$* est un groupe alcoyle ayant un atome de carbone asymétrique et $R_4$ est un groupe alcoyle à chaîne linéaire.

13. Cristal liquide ferroélectrique smectique suivant la revendication 1 de formule

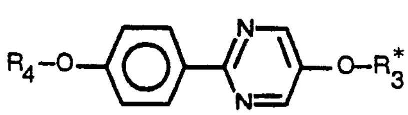

dans laquelle $R_3$* est un groupe alcoyle ayant un atome de carbone asymétrique et $R_4$ est un groupe alcoyle à chaîne linéaire.

14. Cristal liquide ferroélectrique smectique suivant la revendication 1 de formule

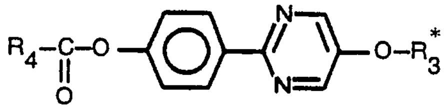

dans laquelle $R_3$* est un groupe alcoyle ayant un atome de carbone asymétrique et $R_4$ est un groupe alcoyle à chaîne linéaire.

15. Additif pour un mélange de cristaux liquides ferroélectriques smectiques de formule développée

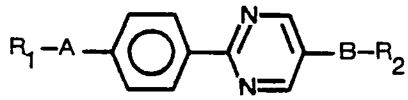

dans laquelle A et B sont respectivement,

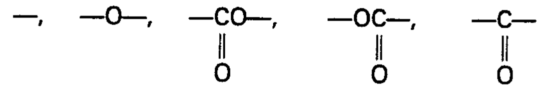

(— est une liaison directe);

l'un de $R_1$ et de $R_2$ est un groupe alcoyle à chaîne linéaire, et l'autre est soit un groupe alcoyle ayant un atome de carbone asymétrique, soit un groupe alcoxyalcoyle ayant un atome de carbone asymètrique et une liaison éther; et

sur l'atome de carbone asymétrique est ajouté l'un des groupes $—CH_3$, —CN, —Cl.

16. Additif pour un mélange de cristaux liquides smectiques suivant la revendication 15, de formule

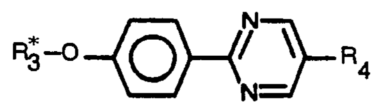

dans laquelle $R_3$* est un groupe alcoyle ayant un atome de carbone asymétrique et $R_4$ est un groupe alcoyle à chaîne linéaire.

17. Additif pour un mélange de cristaux liquides smectiques suivant la revendication 15, de formule

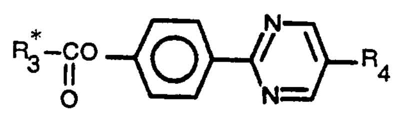

dans laquelle $R_3$* est un groupe alcoyle ayant un atome de carbone asymétrique et $R_4$ est un groupe alcoyle à chaîne linéaire.

18. Additif pour un mélange de cristaux liquides smectiques suivant la revendication 15, de formule

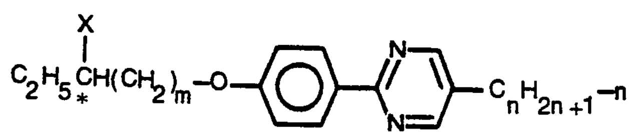

dans laquelle m est un nombre entier de 0 à 8, n est un entier de 5 à 14, X est —Cl ou —CN, C* un atome de carbone asymétrique et le groupe alcoyle contenant n est une chaîne linéaire.

19. Additif pour un mélange de cristaux liquides smectiques suivant la revendication 15, de formule

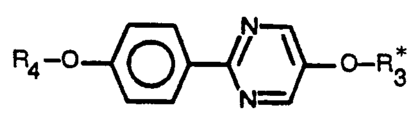

dans laquelle $R_3$* est un groupe alcoyle ayant un atome de carbone asymétrique et $R_4$ est un groupe alcoyle à chaîne linéaire.

20. Additif pour un mélange de cristaux liquides smectiques suivant la revendication 15, de formule

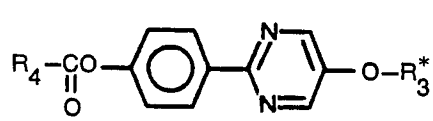

dans laquelle $R_3$* est un groupe alcoyle ayant un atome de carbone asymétrique et $R_4$ est un groupe alcoyle à chaîne linéaire.

FIG. 1

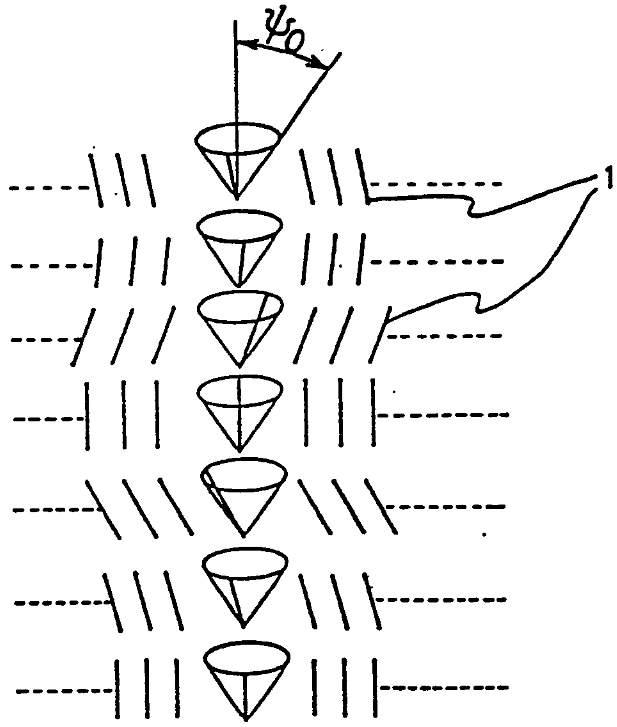


FIG. 2

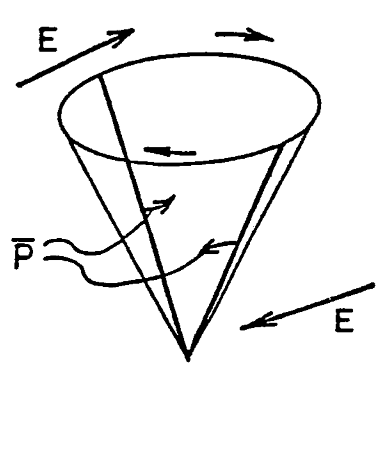

FIG. 3

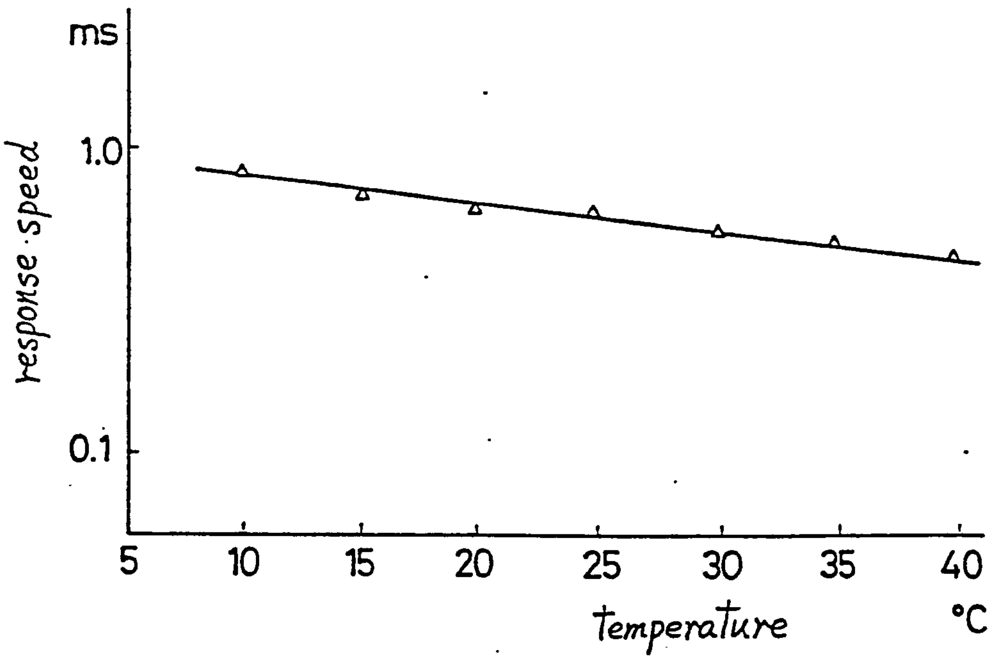

FIG. 4

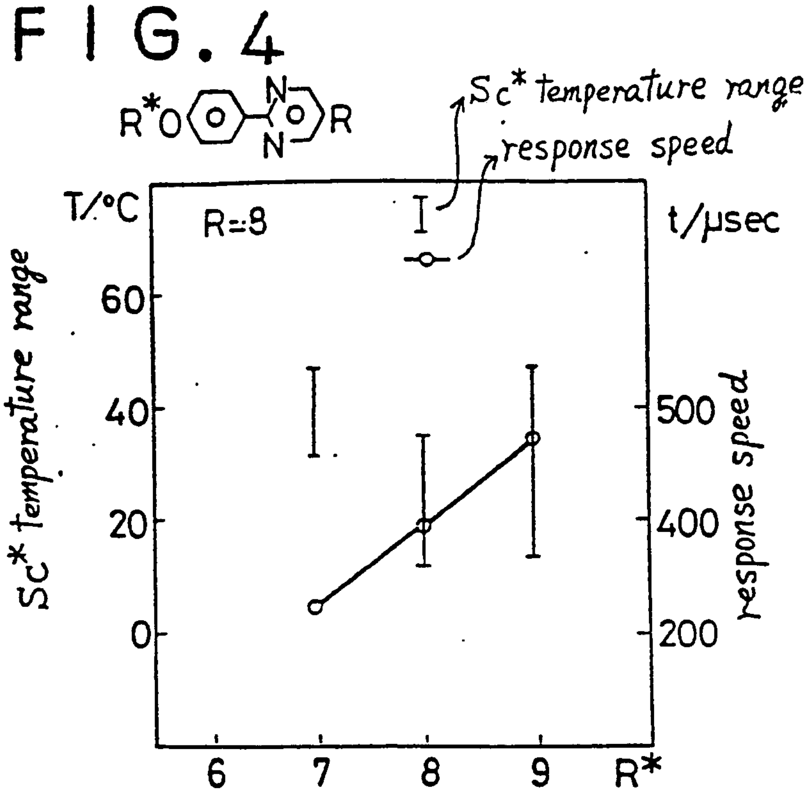
Sc* temperature range
response speed
T/°C
R=8
t/μsec
60
40
20
0
500
400
200
6
7
8
9
R*
Sc* temperature range
response speed

FIG. 5

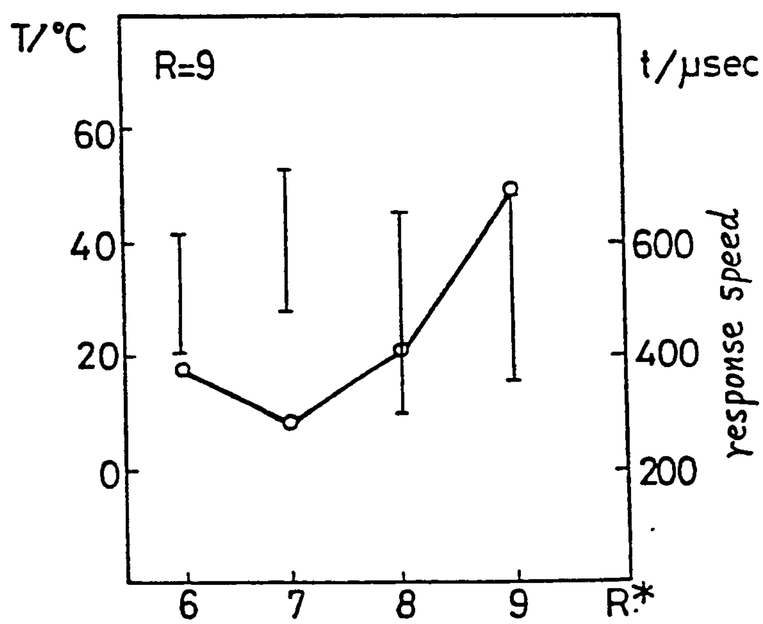
T/°C
R=9
t/μsec
60
40
20
0
600
400
200
6
7
8
9
R*
response speed

FIG. 6

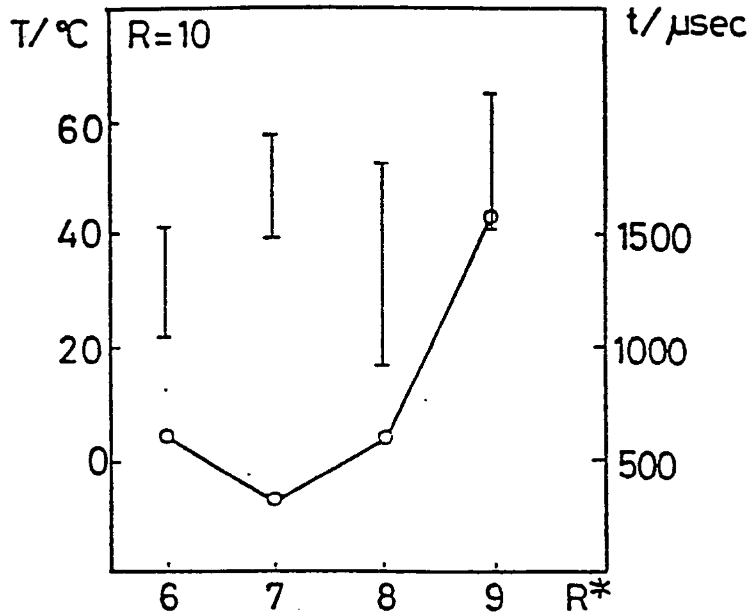
T/°C
R=10
t/μsec
60
40
20
0
1500
1000
500
6
7
8
9
R*

FIG. 7

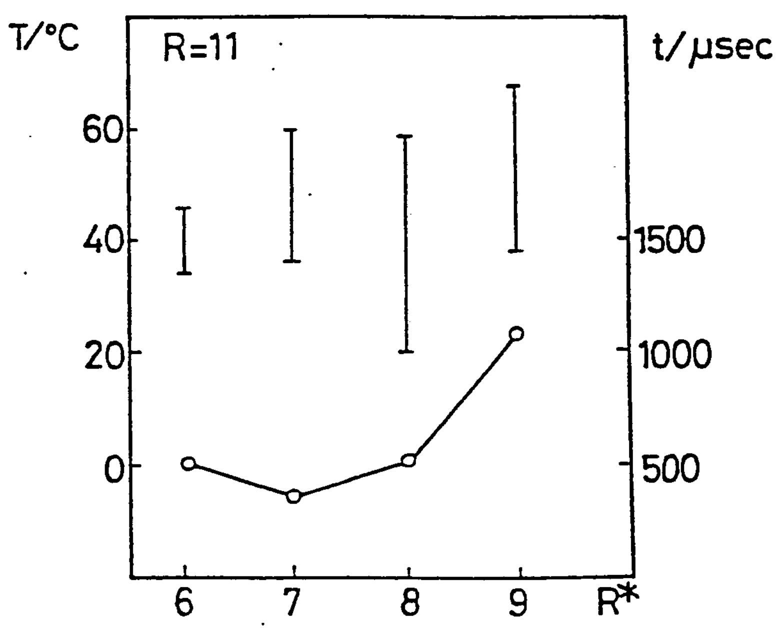
T/°C
R=11
t/μsec
60
40
20
0
1500
1000
500
6
7
8
9
R*

# FIG. 8